(19) Europäisches Patentamt
European Patent Office
Office européen des brevets

(11) **EP 3 761 933 B1**

(12) **EUROPEAN PATENT SPECIFICATION**

(45) Date of publication and mention
of the grant of the patent:
**15.11.2023 Bulletin 2023/46**

(21) Application number: **19763676.4**

(22) Date of filing: **14.02.2019**

(51) International Patent Classification (IPC):
***A61F 13/49*** (2006.01)

(52) Cooperative Patent Classification (CPC):
**A61F 13/49011; A61F 13/49012**

(86) International application number:
**PCT/CN2019/075104**

(87) International publication number:
**WO 2019/169989 (12.09.2019 Gazette 2019/37)**

(54) **WEARABLE ARTICLE COMPRISING AN ELASTIC LAMINATE**

AM KÖRPER TRAGBARER ARTIKEL MIT EINEM ELASTISCHEN LAMINAT

ARTICLE POUVANT ÊTRE PORTÉ COMPRENANT UN STRATIFIÉ ÉLASTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.03.2018 PCT/CN2018/078113**

(43) Date of publication of application:
**13.01.2021 Bulletin 2021/02**

(73) Proprietor: **The Procter & Gamble Company**
**Cincinnati, OH 45202 (US)**

(72) Inventors:
• **CHEN, Meng**
**Beijing 101312 (CN)**
• **GAO, Xu**
**Beijing 101312 (CN)**
• **SHI, Fukuan**
**Shenzhen, Guangdong 518000 (CN)**
• **ERDEM, Gueltekin**
**Beijing 101312 (CN)**
• **MORIMOTO, Koichi**
**Beijing 101312 (CN)**
• **CLAUSSEN, Jan**
**65824 Schwalbach (DE)**

(74) Representative: **P&G Patent Germany**
**Procter & Gamble Service GmbH**
**Sulzbacher Straße 40**
**65824 Schwalbach am Taunus (DE)**

(56) References cited:
WO-A1-2017/002484      WO-A1-2018/118581
WO-A1-2018/118883      CA-A1- 2 158 790
JP-A- 2010 246 901      JP-A- 2017 113 186
US-A1- 2006 047 260      US-A1- 2008 287 897
US-A1- 2010 307 668      US-A1- 2017 119 595
US-A1- 2017 319 403

**Description**

FIELD OF THE INVENTION

The present invention relates to a wearable article comprising an elastic laminate suitable for use in wearable articles which exhibits improved sweat management properties.

BACKGROUND OF THE INVENTION

Substrate materials such as nonwoven fabrics and laminates thereof, are commonly used for wearable articles such as absorbent articles. For example, absorbent articles typically use nonwoven substrate materials for both the skin facing side as well as the garment facing side of the articles, to control the movement of liquids and to provide a comfortable, conforming fit when the article is worn by a wearer. By comfortableness, what may be desired is a cloth-like substrate which is capable of effectively absorbing sweat and excess moisture from the skin and releasing them outside the article. Such is particularly desired for absorbent articles by caregivers of young children, wherein skin health is closely associated with the absence of heat rashes and diaper rashes. Heat rashes in the waist area may be associated with wetness or dampness in the waist area inside an absorbent article. It is a common practice for caregivers to check the degree of wetness or dampness by touching the waist area inside the absorbent article worn by a young child.

Elastic laminates having sweat management properties have been proposed, such as those described in Japanese Patent Application publications 2017-12319A and 2017-113186A. There is a need to provide elastic laminates with further improved sweat management properties, while being economic to make.

JP 2010 246901 relates to a disposable diaper with a hydrophilic sheet arranged at both waist flaps. The hydrophilic sheet includes an inner hydrophilic sheet, and an outer hydrophilic sheet. The outer hydrophilic sheet is more highly liquid diffusible than the inner hydrophilic sheet.

JP 2017 113186 discloses a disposable diaper with abdominal-side waist flaps and back-side waist flaps. Each back-side waist flap includes an outer layer sheet, an inner layer sheet, and a joining part for joining the outer layer sheet and the inner layer sheet. The outer layer sheet has a hydrophilic property, while the inner layer sheet has a hydrophobic property. At a peripheral part of the joining part, hydrophobic fibers of the inner layer sheet enter gaps between hydrophilic fibers of the outer layer sheet, and vice versa.

WO 2017/002484 is concerned with a composite sheet which has a first surface and a second surface. A hydrophobic non-woven fabric forms the first surface and a hydrophilic non-woven fabric forms the second surface. In the composite sheet, a concavo-convex structure is formed on at least the hydrophilic non-woven fabric among the hydrophobic non-woven fabric and the hydrophilic non-woven fabric, and the hydrophobic non-woven fabric and the hydrophilic non-woven fabric are in direct contact with each other at least partially.

US 2006/047260 relates to a disposable absorbent article comprising a chassis which includes a front region having two opposing longitudinal edges; a back region having two opposing longitudinal edges; a crotch region having two longitudinal edges. At least two side panels extend outwardly from the two opposing longitudinal edges of one or both of the front or back regions and interconnect the front and the back regions to form a waist opening and a pair of leg openings.

US 2008/287897 discloses disposable protective undergarments including a front portion and a back portion each having a stretchable waist area, a stretchable leg area, and a stretchable belly area positioned between the waist area and the leg area is provided.

SUMMARY OF THE INVENTION

The present invention is directed to a wearable article comprising an elastic laminate, the elastic laminate comprising

an inner first nonwoven layer and an outer second nonwoven layer, wherein the inner layer and the outer layer are directly joined with each other over an area of from about 5% to about 50%, in that the inner layer and the outer layer are directly secured to each other by applying adhesive agents;
the inner layer being closer to the body of the wearer than the second layer when the article is worn, the inner layer having a first surface facing away from the outer layer and a second surface facing towards the outer layer,
the outer layer having a higher hydrophilicity than the inner layer and having a first surface facing towards the inner layer and a second surface facing away from the inner layer, the inner layer being hydrophobic and the outer layer being hydrophilic;
the laminate having a total caliper when subjected to the NMR MOUSE method as defined herein, the total caliper having a first sub-caliper corresponding to 50 % of the total caliper, starting from the outer layer's second surface and extending towards the inner layer's first surface, and a second sub-caliper corresponding to the remaining 50% of the total caliper, starting from the inner layer's first surface and extending towards the outer layer's second surface, and

wherein the elastic laminate has a Liquid Ratio of the first sub-caliper to the second sub-caliper of at least 1.1, or at least 1.2, or at least 1.3, after 1 minute according to the NMR MOUSE method set out herein, wherein, in the elastic laminate, the basis weight of the inner layer is not greater than the basis weight of the outer layer.

The elastic laminate may comprises an inner layer fold over region wherein the inner layer is elongated beyond the elastic laminate, and the elongated part of the inner layer is folded over itself, the folded over part of the inner layer thereby forming a third layer such that at least a portion of the elastic laminate comprises the inner, outer and third layer (the inner layer being sandwiched between the third layer and the outer layer). The third layer may be attached to the first surface of the inner layer, e.g. by adhesive such as intermittent adhesive bonding. The third layer facing towards the skin of the wearer in use may be treated to have higher hydrophobicity than the inner layer.

In addition, the elastic laminate may further comprise an outer layer fold over region wherein the outer layer is elongated beyond the elastic laminate and the elongated part of the outer layer is folded over the third layer, the folded over part of the outer layer thereby forming a fourth layer such that at least a portion of the elastic laminate comprises the inner layer and third layer sandwiched between outer layer and the fourth layer. The fourth layer may be attached to the third layer, e.g. by adhesive such as intermittent adhesive bonding. The third layer may extend beyond the fourth layer (i.e. cover a larger surface area than the fourth layer). The extension of the fourth layer may form less than 20%, or less than 10% of the surface area of the elastic laminate, determined when the elastic laminate is stretched such that the inner and outer layer are flattened out.

Alternatively, The elastic laminate may comprise only an outer layer fold over region (i.e. no inner layer fold over region), wherein the outer layer is elongated beyond the elastic laminate, and the elongated part of the outer layer is folded over the elastic laminate, the folded over part of the outer layer thereby forming a third layer such that at least a portion of the elastic laminate comprises the inner layer sandwiched between the outer layer and the third layer. The third layer may be attached to the first surface of the inner layer, e.g. by adhesive such as intermittent adhesive bonding. The third layer facing towards the body of the wearer in use may be treated to have equal or higher hydrophobicity than the inner layer.

Of course, in a still further alternative, the elastic laminate may not comprise any folded over extensions of the inner and outer layer at all.

At least 40%, or at least 50% of the first surface of the inner layer, and/or at least 50% of the third layer formed by folding over an extension of the inner layer may be in direct contact with the body of the wearer during use of the wearable article.

The elastic laminate may form an elastic belt of the wearable article, the elastic belt comprising a front belt and a rear belt. The first surface, or at least 50% of the first surface, of the inner layer may be in direct contact with the body of the wearer during use of the wearable article.

The elastic laminate defined above enables fast and reliable transport of liquid, such as sweat, away from the body of the wearer and through the elastic laminate to the outside.

Liquid transport away from the skin and through the laminate is measured by using NMR MOUSE (Nuclear Magnetic Resonance Mobile Universal Surface Explorer) methodology. NMR MOUSE is a portable device using an open NMR sensor to characterize fluid positioning inside a porous media structure (i.e. the elastic laminate). The method enables precise determination of liquid distribution at different points in time.

For the method, the inner layer of the elastic laminate is brought into contact with a solution of 0.9% sodium chloride (see detailed method description below) and a weight of 0.25 psi is applied onto the second surface of the outer layer to simulate normal in use conditions when a standing person wears the wearable article. A Liquid Ratio of the first sub-caliper to the second sub-caliper of at least 1.1 after 1 minute means that, already 1 minute after the elastic laminate has been brought into contact with the liquid solution, more than 50% of the liquid within the elastic laminate has already been distributed to the first sub-caliper of the elastic laminate which is facing away from the body of the wearer.

Having an elastic laminate comprising an inner and an outer layer, it is essential that liquid moves quickly from the inner layer to and through the outer layer to enable efficient sweat management. Elastic laminates having a Liquid Ratio of the first sub-caliper to the second sub-caliper of at least 1.1, or at least 1.2, or at least 1.3 after 1 minute according to the NMR MOUSE method provide good sweat management, thus contributing to improved skin health.

The elastic laminate may have a Liquid Ratio of the first sub-caliper to the second sub-caliper of at least 1.5, or at least 1.8 after 10 minutes, and of at least 2.0 after 20 minutes according to the NMR MOUSE method provide good sweat management, thus contributing to improved skin health.

BRIEF DESCRIPTION OF THE DRAWINGS

Figures 1A-1B are a schematic plan views of one embodiment of a wearable article of the present invention with the seams un-joined and in a flat uncontracted condition showing the garment facing surface.
Figures 2A-2C are schematic cross section views of embodiments of wearable articles of the present invention.
Figures 3 shows a schematic representation of an NMR sensor.
Fig. 4 shows a schematic representation of a specimen and equipment as prepared for and placed on the NMR MOUSE sensor.
Fig. 5A shows the determination of the total Caliper and first and second sub-caliper of Specimen from Wet Distribution Profile for Comparative Example 5.
Fig. 5B shows the determination of Sample Holder Surface from Distribution Profile for Comparative Example 5 (graph shows NMR profile when sample is not inserted).
Fig. 6 shows the Dry Distribution Profile and Wet Distribution Profile (after 1 minute and 20 minutes) of Example 1.
Fig. 7 shows the Dry Distribution Profile and Wet Distribution Profile (after 1 minute and 20 minutes) of Example 2.
Fig. 8 shows the Dry Distribution Profile and Wet Distribution Profile (after 1 minute and 20 minutes) of Example 3.
Fig. 9 shows the Dry Distribution Profile and Wet Distribution Profile (after 1 minute and 20 minutes) of Example 4.
Fig. 10 shows the Dry Distribution Profile and Wet Distribution Profile (after 1 minute and 20 minutes) of Example 5.
Fig. 11 shows the Dry Distribution Profile and Wet Distribution Profile (after 1 minute and 20 minutes) of Example 6.
Fig. 12 shows the Dry Distribution Profile and Wet Distribution Profile (after 1 minute and 20 minutes) of Comparative Example 1.
Fig. 13 shows the Dry Distribution Profile and Wet Distribution Profile (after 1 minute and 20 minutes) of Comparative Example 4.
Fig. 14 shows the Dry Distribution Profile and Wet Distribution Profile (after 1 minute and 20 minutes) of Comparative Example 5.
Fig. 15 shows the Dry Distribution Profile and Wet Distribution Profile (after 1 minute and 20 minutes) of Comparative Example 7.
Fig. 16 shows the Dry Distribution Profile and Wet Distribution Profile (after 1 minute and 20 minutes) of Comparative Example 8.
Fig. 17 shows the Dry Distribution Profile and Wet Distribution Profile (after 1 minute and 20 minutes) of Comparative Example 9.

DEFINITIONS

As used herein, the following terms shall have the meaning specified thereafter:
"Wearable article" refers to articles of wear which may be in the form of pants, taped diapers, incontinent briefs, feminine hygiene garments, wound dressings, hospital garments, and the like. Preferably, the wearable article of the present invention is a pant. The "wearable article" may be so configured to also absorb and contain various exudates such as urine, feces, and menses discharged from the body. The "wearable article" may serve as an outer cover adaptable to be joined with a separable disposable absorbent insert for providing absorbent and containment function, such as those disclosed in PCT publication WO 2011/087503A.
As used herein, " taped diaper" and "pant" refers to an absorbent article generally worn by babies, infants and incontinent persons about the lower torso to encircle the waist and legs of the wearer and that is specifically adapted to receive and contain urinary and fecal waste. In a pant, as used herein, the longitudinal edges of the first and second waist region are attached to each other to a pre-formed waist opening and leg openings. A pant is generally placed in position on the wearer by inserting the wearer's legs into the leg openings and sliding the pant absorbent article into position about the wearer's lower torso. A pant may be pre-formed by any suitable technique including, but not limited to, joining together portions of the absorbent article using refastenable and/or non-refastenable bonds (i.e. with permanent side seams not intended to be torn upon prior to removal of the pant from the wearer for disposal). In a diaper, the waist opening and leg openings are only formed when the diaper is applied onto a wearer by (releasable) attaching the longitudinal edges of the first and second waist region to each other on both sides by a suitable fastening system.
"Taped diaper" refers to disposable absorbent articles which are applied on a wearer by tapes.
As used herein, "disposable" is used in its ordinary sense to mean an article that is disposed or discarded after a limited number of usage over varying lengths of time, for example, less than 20 usages, less than 10 usages, less than 5 usages, or less than 2 usages. If the disposable absorbent article is a taped diaper, a pant, sanitary napkin, sanitary pad or wet wipe for personal hygiene use, the disposable absorbent article is most often intended to be disposed after single use. The absorbent articles described herein are disposable.

"Longitudinal" refers to a direction running substantially perpendicular from a waist edge to an opposing waist edge of the article and generally parallel to the maximum linear dimension of the article. "Transverse" refers to a direction perpendicular to the longitudinal direction.

"Inner" and "outer" refer respectively to the relative location of an element or a surface of an element or group of elements. "Inner" implies the element or surface is nearer to the body of the wearer during wear than some other element or surface. "Outer" implies the element or surface is more remote from the body of the wearer during wear than some other element or surface (i.e., element or surface is more proximate to the wearer's garments that may be worn over the present article).

"Body-facing" and "garment-facing" refer respectively to the relative location of an element or a surface of an element or group of elements. "Body-facing" implies the element or surface is nearer to the wearer during wear than another element of the same component. An example is the inner layer of the elastic laminate of the present invention wherein the inner layer (being an element of the elastic laminate) is nearer to the body of the wearer than the outer layer (being another element of the elastic laminate). "Garment-facing" implies the element or surface is more remote from the wearer during wear than another element of the same component. The garment-facing surface may face another (i.e. other than the wearable article) garment of the wearer, other items, such as the bedding, or the atmosphere.

"Proximal" refers to a portion being closer relative to the longitudinal center of the article, while "distal" refers to a portion being farther from the longitudinal center of the article.

"Film" refers to a sheet-like material wherein the length and width of the material far exceed the thickness of the material. Typically, films have a thickness of about 0.5 mm or less.

A "nonwoven layer" is a nonwoven material which is a manufactured web of directionally or randomly oriented fibers, consolidated and bonded together, e.g. by one or more patterns of bonds and bond impressions created through localized compression and/or application of heat or ultrasonic energy, or a combination thereof. The term "nonwoven" does not include fabrics which are woven, knitted, or stitch-bonded with yarns or filaments. The fibers may be of natural or man-made origin and may be staple or continuous filaments. Nonwoven layers can be formed by many processes such as meltblowing, spunbonding, solvent spinning, electrospinning, and carding. Often, a nonwoven layer consists of sub-layers, which may be spunbond or meltblown, such as SMS, SMMS or the like. An SMS nonwoven layer consists of inner and outer spunbond sub-layers with a meltblown layer in between. In such nonwoven layer, the sub-layers are laid down on top of each other and the nonwoven layer is subsequently consolidated e.g. by thermal point (combination of heat and pressure in dedicated bond points) bonding to form a coherent nonwoven layer. The steps of laying down the sub-fibers and nonwoven layer consolidation are often carried out on one continuous manufacturing line. The basis weight of nonwoven fabrics is usually expressed in grams per square meter (g/m$^2$). A nonwoven layer may be consolidated and bonded by hydroentanglement and/or needle punching, in addition to being consolidated and bonded by bonds obtained by heat and/or compression (including ultrasonic bonding). Carded nonwoven layers are formed of short, so-called staple fibers. They are typically formed into a layer of fibers and subsequently consolidated into a nonwoven layer, for example by autogenously bonding the fibers together with heat and/or by intertwining the fibers by known processes such as hydroentangling or needle-punching. The carded fibers may also be bonded together, e.g. by one or more patterns of bonds and bond impressions created through localized compression and/or application of heat or ultrasonic energy, or a combination thereof.

"Water-permeable" and "water-impermeable" refer to the penetrability of materials in the context of the intended usage of disposable absorbent articles. Specifically, the term "water-permeable" refers to a layer or a layered structure having pores, openings, and/or interconnected void spaces that permit liquid water, urine, or synthetic urine to pass through its thickness in the absence of a forcing pressure. Conversely, the term "water-impermeable" refers to a layer or a layered structure through the thickness of which liquid water, urine, or synthetic urine cannot pass in the absence of a forcing pressure (aside from natural forces such as gravity). A layer or a layered structure that is water-impermeable according to this definition may be permeable to water vapor, i.e., may be "vapor-permeable".

"Hydrophilic" describes surfaces of substrates which are wettable by aqueous fluids (e.g., aqueous body fluids) deposited on these substrates. Hydrophilicity and wettability are typically defined in terms of contact angle and the strike-through time of the fluids, for example through a nonwoven fabric. This is discussed in detail in the American Chemical Society publication entitled "Contact Angle, Wettability and Adhesion", edited by Robert F. Gould (Copyright 1964). A surface of a substrate is said to be wetted by a fluid (i.e., hydrophilic) when either the contact angle between the fluid and the surface is less than 90°, or when the fluid tends to spread spontaneously across the surface of the substrate, both conditions are normally co-existing. Conversely, a substrate is considered to be "hydrophobic" if the contact angle is greater than 90° and the fluid does not spread spontaneously across the surface of the fiber.

"Extendibility" and "extensible" mean that the width or length of the component in a relaxed state can be extended or increased.

"Elasticated" and "elasticized" mean that a component comprises at least a portion made of elastic material.

"Elongation rate" means the state of elongation of a material from its relaxed, original length, namely an elongation rate of 10% means an elongation resulting in 110% of its relaxed, original length.

"Elongatable material", "extensible material", or "stretchable material" are used interchangeably and refer to a material that, upon application of a biasing force, can stretch to an elongation rate of at least 10% (i.e. can stretch to 10 percent more than its original length), without rupture or breakage, and upon release of the applied force, shows little recovery, less than about 20% of its elongation without complete rupture or breakage as measured by EDANA method 20.2-89. In the event such an elongatable material recovers at least 40% of its elongation upon release of the applied force, the elongatable material will be considered to be "elastic" or "elastic." For example, an elastic material that has an initial length of 100mm can extend at least to 150mm, and upon removal of the force retracts to a length of at least 130mm (i.e., exhibiting a 40% recovery). In the event the material recovers less than 40% of its elongation upon release of the applied force, the elongatable material will be considered to be "substantially non-elastic" or "substantially non-elastic". For example, an elongatable material that has an initial length of 100mm can extend at least to 150mm, and upon removal of the force retracts to a length of at least 145mm (i.e., exhibiting a 10% recovery).

DETAILED DESCRIPTION OF THE INVENTION

Elastic laminate of the wearable article:

The elastic laminate of the present invention comprises an inner layer being a nonwoven and an outer layer being nonwoven having higher hydrophilicity than the inner layer. The inner layer has a first surface facing away from the outer layer, and a second surface facing towards the outer layer. Hence, the first surface of the inner layer faces towards the skin of the wearer when the article is in use. The outer layer has a first surface facing towards the inner layer, and second surface facing away from the inner layer. Hence, the second surface of the outer layer faces towards the garment (overlaying the wearable article) of the wearer when the article is in use.

The inner and outer layer are directly joined with each other over an area of from about 5% to about 50%. By "directly joined" what is meant is that the inner layer and the outer layer are directly secured to each other by applying adhesive agents. The percentage of area of the inner and outer layers that are directly joined with each other may vary depending on the joining method for forming the elastic laminate, as discussed in further detail below. The inner and outer layers are directly joined with each other over an area of from about 5% to about 50% to provide the sweat management property, while also maintaining integrity as an elastic laminate. The relationship of the outer layer having higher hydrophilicity than the inner layer comprises situations where the inner layer is hydrophobic and the outer layer is hydrophilic. The inner and outer layers may have a difference in contact angle of at least about 10 degrees, or a difference in contact angle of at least 15 degrees, or a difference in contact angle of at least 20 degrees. Without being bound by theory, it is believed that, by providing the inner layer relatively more hydrophobic, the outer layer relatively more hydrophilic, effective liquid removal and transport to the outer layer is facilitated.

The outer layer may have a plurality of openings at an Opening Rate of from about 5% to about 50% according to the measurements herein. By further provide a certain opening area for the outer layer, there is provided multiple moisture transport channels are provided which can contribute to an effective liquid removal and transport to the outer layer. The transport channels may be driven by capillary force gradient, and enhanced exposure to outside the laminate away from the skin.

Further, the relationship of the inner layer and the outer layer with regards: fiber diameter, porosity, void cell diameter, thickness, and basis weight; may further play a role in effective liquid removal and transport. Porosity may be measured directly, or may be evaluated indirectly by measuring the Mean Flow Pore Size based on capillary flow porometry as described in the test method below. The inner layer may have a Mean Flow Pore Size of from about 50 $\mu$m to about 250 $\mu$m, or from about 55 $\mu$m to about 120 $\mu$m, or from about 60 $\mu$m to about 170 $\mu$m, or from about 65 $\mu$m to about 220 $\mu$m, or from about 70 $\mu$m to about 240 $\mu$m. The outer layer may have a Mean Flow Pore Size of from about 100 $\mu$m to about 400 $\mu$m, or from about 105 $\mu$m to about 350 $\mu$m, or from about 110 $\mu$m to about 300 $\mu$m, or from about 115 $\mu$m to about 250 $\mu$m. Optionally providing the outer layer with a plurality of openings at an Opening Rate of from about 5% to about 50% may contribute in providing the thickness gradient.

Also, to provide a thickness gradient, the basis weight of the inner layer is not greater than the basis weight of the outer layer. The inner layer of the present invention is a nonwoven which may have a basis weight of from about 5g/m$^2$ to about 45g/m$^2$, or from about 5g/m$^2$ to about 35g/m$^2$. The inner layer nonwoven may have a fiber diameter of from about 0.5 dpf to about 4 dpf. The fiber diameter is described in denier per filament (dpf) used in the industry, which is grams / 9,000 meters of length of fiber. The inner layer nonwoven may be made by processes such as spunbond, spunlace, carded or air-laid; and may comprise fibers and/or filaments made of polypropylene (PP), polyethylene (PE), polyethylene phthalate (PET), polylactic acid/polylactide (PLA) or conjugate fibers (such as PE/PET, PE/PP, PE/PLA) as well as natural fibers such as cotton or regenerated cellulosic fibers such as viscose or lyocell. The inner layer nonwoven may also be a multilayer or composite structure combining nonwovens made by different processes and fibers such as combining spunbond and carded nonwovens. The inner layer nonwoven may be made by biodegradable material, or derived from renewable resources. Non-limiting examples of materials suitable for the inner layer nonwoven of the present invention include: 12-30gsm air-through carded nonwoven substrate made of PE/PET bi-component staple fiber, such as those available from Beijing Dayuan Nonwoven Fabric Co. Ltd. or Xiamen Yanjan New Material Co. Ltd., and 8-30gsm spun melt nonwoven substrate comprising PP monofilament or PE/PP bi-component fibers, such as those available from Fibertex or Fitesa.

The inner layer nonwoven may be inherently hydrophobic. The inner layer nonwoven may be provided hydrophobicity by treating with hydrophobic melt additives into polymer resin in the fiber making process, or applying hydrophobic additives after the nonwoven is formed.

Hydrophobic additives may be fatty acids originated from vegetable, animal, and/or synthetic sources. Fatty acids may range from C8-C30 fatty acid, or from C12-C22 fatty acid, or substantially saturated fatty acid. Hydrophobic additives may be fatty acid derivatives including fatty alcohols, fatty acid esters, and fatty acid amides. Suitable fatty alcohols include those derived from C12-C30 fatty acids. Suitable fatty acid esters include those fatty acid esters derived from a mixture of C12-C30 fatty acids and short chain monohydric alcohols, preferably from a mixture of C12-C22 saturated fatty acids and short chain monohydric alcohols. The hydrophobic melt additive may comprise a mixture of mono, di, and/or tri-fatty acid esters. An example includes fatty acid ester with glycerol having at least one alkyl chain, at least two, or three chains to a glycerol, to form a mono, di, or triglyceride. Suitable triglycerides include glycerol thibehenate, glycerol tristearate, glycerol tripalmitate, and glycerol trimyristate, and mixtures thereof. Exemplary hydrophobic melt additives include glyceryl tristearate, such as those commercially available as Techmer PPM15000. Hydrophobic agents may be fatty acid amides including those derivatives from a mixture of C12-C28 fatty acids (saturated or unsaturated) and primary or secondary amines such as erucamide, oleamide and behanamide.

Exemplary hydrophobic additives which may be applied after the nonwoven is formed include surfactants and silicone-based finishes, natural oil or wax such as cotton seed oil, beeswax and shea butter.

The inner layer may optionally have a plurality of openings at an Opening Rate of from about 5 % to about 30 %, or from about 5 % to about 15 %, or from about 6 % to about 8 %, or from about 7 % to about 15 %, or from about 9 % to about 25 %, and an Effective Opening Area of from about 0.1 mm$^2$ to about 25 mm$^2$, or from about 0.1 mm$^2$ to about 10 mm$^2$, or from about 0.4 mm$^2$ to about 2.0 mm$^2$, or from about 0.5 mm$^2$ to about 4 mm$^2$, of from about 1.0 mm$^2$ to about 5 mm$^2$, or from about 4.0 mm$^2$ to about 8 mm$^2$, or from about 7 mm$^2$ to about 15 mm$^2$, according to the measurements herein.

The outer layer of the present invention is a nonwoven which may have a basis weight of from about 10g/m$^2$ to about 45g/m$^2$, or from about 10g/m$^2$ to about 35g/m$^2$, and may be adjusted such that the basis weight of the inner layer is not greater than the basis weight of the outer layer. Without being bound by theory, by providing the basis weight relationship as such, it is believed that skin sweating is effectively transported to the outer layer and outside the laminate, while preventing the transported sweat back to the inner layer. The outer layer nonwoven may have a fiber diameter of from about 0.8 dpf to about 6 dpf. The outer layer nonwoven may be made by processes such as spunbond, spunlace, carded or air-laid; and may comprise fibers and/or filaments made of polypropylene (PP), polyethylene (PE), polyethylene phthalate (PET), polylactic acid/polylactide (PLA) or conjugate fibers (such as PE/PET, PE/PP, PE/PLA) as well as natural fibers such as cotton or regenerated cellulosic fibers such as viscose or lyocell. The outer layer nonwoven may also be a multilayer or composite structure combining nonwovens made by different processes and fibers such as combining spunbond and carded nonwovens. The outer layer nonwoven may be made by biodegradable material, or derived from renewable resources.

The outer layer nonwoven may inherently be hydrophobic, and thus provided relatively more hydrophilic than the inner layer by treating with hydrophilic melt additives into polymer resin in the fiber making process, or applying hydrophilic additive after the nonwoven is formed.

Hydrophilic additives may be polypropylene and polyethylene polymers such as those available from Techmer PM (Clinton, TN, US) sold under the trade name of Techmer PPM15560; TPM12713, PPM19913, PPM 19441, PPM19914, and PM19668. Hydrophilic additives may include, ionic surfactants, cationic surfactants, amphoteric surfactants or mixtures thereof. Exemplary hydrophilic additives include 100410 AF PE MB marketed by Ampacet, Irgasuf HL560 commercially available from Ciba Speciality Chemicals Inc., Hydrosorb 1001 commercially available from Goulston Technologies Inc., Cirrasol PP682 commercially available from Uniqema, Stantex S 6327 commercially available from Cognis, Silastol PST, Silastol PHP26 commercially available from Schill & Seilacher, Silwet L-7608 commercially available from Momentive Performance Materials, silicone surfactant with a polyethylene oxide chain and molecular weight above 700 g/mol by the name Polyvel S-1416 or VW 315 commercially available from Polyvel Inc.

Exemplary material for the outer layer include: air-through carded nonwoven having a thickness of at least about 50 μm, or at least about 80 μm, or at least about 200 μm. The thickness may be less than 2000 μm, or less than 1500 μm, or less than 1250 μm. Such material may provide a soft lofty feeling to the garment-facing layer. Suitable for the outer layer nonwoven of the present invention are air-through carded nonwoven material made of co-centric bicomponent fiber, crimping fiber made through core eccentric bicomponent filament or side by side bicomponent filament. Non-limiting examples of materials suitable for the outer layer nonwoven of the present invention include: 12 g/m$^2$ to 45g/m$^2$ air-through carded nonwoven substrate comprising PE/PET bi-component fibers, such as those available from Beijing Dayuan Nonwoven Fabric Co. Ltd. or Xiamen Yanjan New Material Co. Ltd., and 8-45gsm spun melt nonwoven substrate comprising PP monofilament or PE/PP bi-component fibers, such as those available from Fibertex or Fitesa.

The basis weight and material thickness of the inner and outer nonwoven layer herein is related to materials obtained from a finished product according to the "Preparation for Thickness and Basis Weight" below and measured by "Base caliper method - ASTM D 654 Standard Test Method for Thickness of Paper and Paper Board" with modification of the loading to 500Pa, and by "Basis weight - ASTM D 756 Practice for Determination of Weight and Shape Changes of Plastics Under Accelerated Service Conditions", respectively. Total caliper of the elastic laminate is determined in the NMR MOUSE test method set out below.

The outer layer may have a plurality of openings at an Opening Rate of from about 5% to about 50%, or from about 5% to about 40%, or from about 5% to about 30%, or from about 6% to about 8%, or from about 7% to about 15%, or from about 9% to about 25%, and an Effective Opening Area of from about $0.1mm^2$ to about $25mm^2$, or from about $0.1mm^2$ to about $10mm^2$, or from about $0.4mm^2$ to about $2.0 mm^2$, or from about $0.5mm^2$ to about $4mm^2$, of from about $1.0 mm^2$ to about $5 mm^2$, or from about $4.0 mm^2$ to about $8 mm^2$, or from about $7 mm^2$ to about $15 mm^2$, according to the measurements herein.

Alternatively, or in addition, the inner layer may haves a plurality of openings at an Opening Rate of from about 5% to about 50%, or from about 5% to about 40%, or from about 5% to about 30%, or from about 6% to about 8%, or from about 7% to about 15%, or from about 9% to about 25%, and an Effective Opening Area of from about 0.1mm2 to about 25mm2, or from about 0.1mm2 to about 10mm2, or from about 0.4mm2 to about 2.0 mm2, or from about 0.5mm2 to about 4mm2, of from about 1.0 mm2 to about 5 mm2, or from about 4.0 mm2 to about 8 mm2, or from about 7 mm2 to about 15 mm2, according to the measurements herein.

For either the outer layer and/or the inner layer, the openings may be apertures, slits, or the like. Preferably, the openings are apertures. If the inner and outer layer have openings, the openings of the inner layer may be congruent with the openings of the outer layer. Alternatively, if the inner and outer layer have openings, the openings of the inner layer may not be congruent with the openings of the outer layer. In a still further alternative, if the inner and outer layer have openings, some of the openings in the inner layer may be congruent with the openings in the outer layer, while the remaining openings in the inner layer may not be congruent with the openings of the outer layer.

The openings in the inner and/or outer layer may be apertures having an aspect ratio of less than about 5. The aspect ratio of an opening is determined as such. The greatest dimension of the opening is measured, wherein the direction of the greatest dimension defines the first axis. The line perpendicular to the first axis is defines the second axis. The dimension of the opening along the second axis is measured, and defined the cross dimension. The aspect ratio is the greatest dimension divided by the cross dimension.

The openings may be made by female-male hot pin process, hole punching process, hydroentanglement process using water jets and a screen to create holes, and combinations thereof. The openings may be made by creating a plurality of weakened locations by heat or pressure followed by incrementally stretching, causing said nonwoven web to rupture at the weakened locations such as described in US Patent 5,628,097. Such rupturing method may be particularly useful for nonwovens using spunbonded fibers and meltblown fibers. The openings may be three-dimensional, nonhomogeneous, unaligned and forming a pattern as described in PCT Publication WO 2016/73712.

In the present invention, the openings from the inner layer and the outer layer may be configured to completely match, or partially match, or not match at all. The inner layer or the outer layer may be devoid of openings (alternatively, both layers may be devoid of openings). Namely, the combination of the inner layer and the outer layer may provide a Relative Opening Rate of 100%, less than 100%, or less than about 50%, or less than about 15% according to the measurements herein. What is meant by Relative Opening Rate is the percentage of opening of the elastic laminate which matches the opening of the outer layer. When there is only opening in the outer layer, the Relative Opening Rate is 0%, while when the openings of the outer layer and the inner layer completely match, the Relative Opening Rate is 100%. In the present invention, the inner layer may optionally have openings, and when so, the pattern and density of the openings may be changed so as to completely or partially match with those of the outer layer. Without being bound by theory, in one embodiment, it is believed that, by having at least some portions of some of the openings of the outer layer closed by the inner layer, this provides a liquid permeability gradient from the inner layer to the outer layer, thus sweat is effectively wicked and moved to the outer layer. Without being bound by theory, in another embodiment, it is believed that, by having the openings of the outer layer and the inner layer completely match, this provides favorable breathability and appearance.

The inner layer and the outer layer are directly joined to each other over an area of from about 5% to about 50% by applying adhesive, for providing the elastic laminate of the present invention. The inner and outer layers are at least partially directly joined by adhesive agent. When adhesive agent is used for joining the inner and outer layers, the area in which adhesive agent is applied between the inner and outer layers is considered as area in which the layers are directly joined. When using adhesive agent as a joining means, the adhesive agent may be applied intermittently, such as in spiral pattern. Alternatively or additionally, the adhesive agent may be applied by a slot coat pattern for sake of better process control, wherein the area in which adhesive agent is applied is from about 5% to about 50%, or from about 5% to about 40%, or from about 5% to about 30% of the laminate planar area.

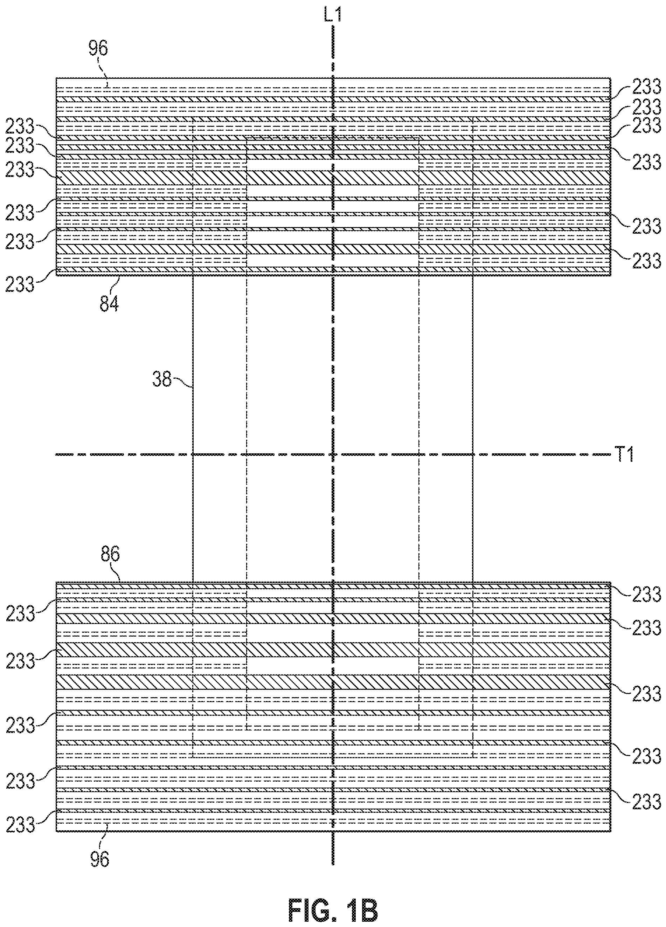

The elastic laminate of the present invention may have an elongation rate of at least about 110% in at least one direction. Elasticity may be imparted by laminating an elastic body between the inner layer and the outer layer. The elastic body may be a plurality of elastic strands or elastic ribbons, or an elastic sheet. The layers and elastic bodies may be at least partially joined by means selected from the group consisting of; adhesive agent, heat, pressure, ultrasound, and combinations thereof. Referring to Figure 1B, adhesive agent may be applied for joining the elastic bodies to the outer and/or inner layer, and further applied via a slot coat in a pattern of panel adhesive agents 233 for joining the outer layer and the inner layer. Alternatively or additionally, the elastic bodies may be joined by deforming the inner and/or outer layer contacting the elastic body via ultrasound or heat, to anchor the elastic body against the inner and/or outer layer. The elastic body may be an elastic sheet; wherein ultrasound is applied at a certain energy level to the inner layer, outer layer, and elastic sheet, combined, such that the fibers of the inner layer and the outer layer come into direct contact with each other. These directly joined areas of fibers are also considered as area in which the inner and outer layers are directly joined.

Elastic laminates obtained by any of the aforementioned joining methods need not be embossed, or mechanically activated, beyond the force needed to at least partially directly join the layers. Thus, the elastic laminate may be economically made. The directly joined area may be measured by stretching the elastic laminate to an uncontracted condition, suitably with a force of 25N, and observing the planar area where the inner and outer layers are directly joined.

The wearable article

The present invention relates to a wearable article comprising an elastic laminate. The elastic laminate may form at least a part of a wearable article that is in direct contact with the skin.

The elastic laminate comprises an inner nonwoven layer and an outer nonwoven layer. The inner nonwoven layer is closer to the wearer than the outer layer when the article is worn. The inner nonwoven layer may be in direct contact with the skin of the wearer when the article is worn. The outer nonwoven layer may form at least a part of the outer surface of the wearable article.

The elastic laminate may be used as a component selected from the group consisting of elastic belts, waistbands, side panels, leg cuffs, and outer covers, of the wearable article.

The present elastic laminate is particularly useful as an elastic belt. The wearable article may be a pant. An exemplary pant is described in PCT Publication WO 2006/17718A. The pant may comprise a central chassis 38 to cover the crotch region of the wearer when the article is worn, a front belt 84 and a back belt 86 (hereinafter may be referred to as "front and back belts") comprising the elastic laminate of the present invention, the front and back belts 84, 86 forming a discrete ring-like elastic belt 40 (hereinafter may be referred to as "waist belt") extending transversely defining the waist opening. The wearable article 20 may be a uni-body type pant wherein the central chassis 38 is continuous with the front and back belt 84, 86, wherein the leg openings are continuously formed (not shown). The belt-type pant may be advantageous in that the central chassis 38 has better breathability, thus providing better sweat management for the entire wearable article.

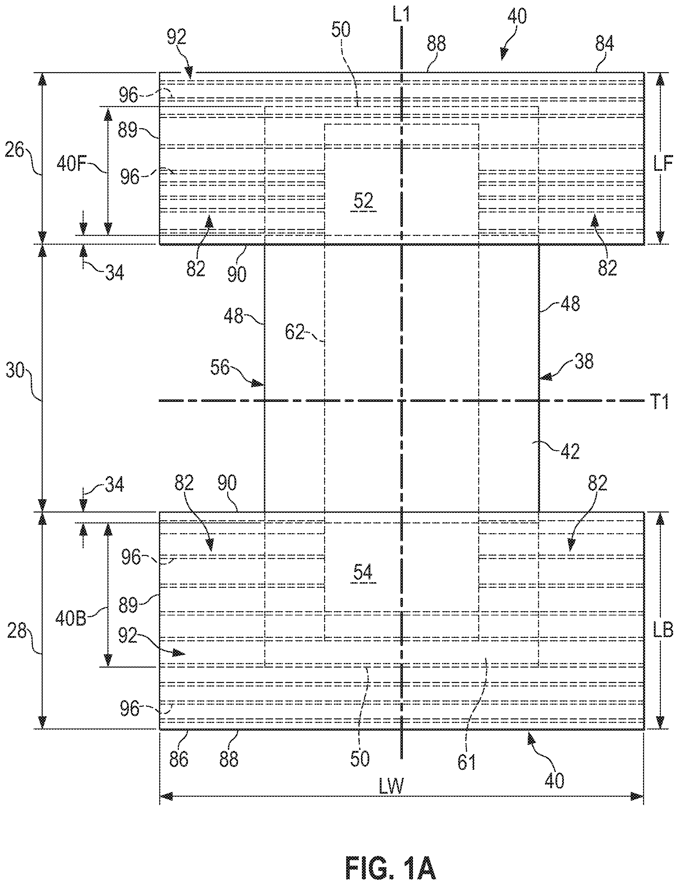

Figure 1A is a perspective view of an example for a wearable article of the present invention of the pant with the seams un-joined and in its flat uncontracted condition showing the garment-facing surface. The wearable article has a longitudinal centerline L1 which also serves as the longitudinal axis, and a transverse centerline T1 which also serves as the transverse axis. The wearable article has a body facing surface, a garment facing surface, a front region 26, a back region 28, a crotch region 30, and seams which join the front region 26 and the back region 28 to form two leg openings and a waist opening. At least a portion of or the entirety of the front belt 84, or at least a portion of or the entirety of the back belt 86, or the entire discrete ring-like elastic belt 40 may be made by the elastic laminate of the present invention. The front and back belts 84, 86 and the central chassis 38 jointly define the leg openings.

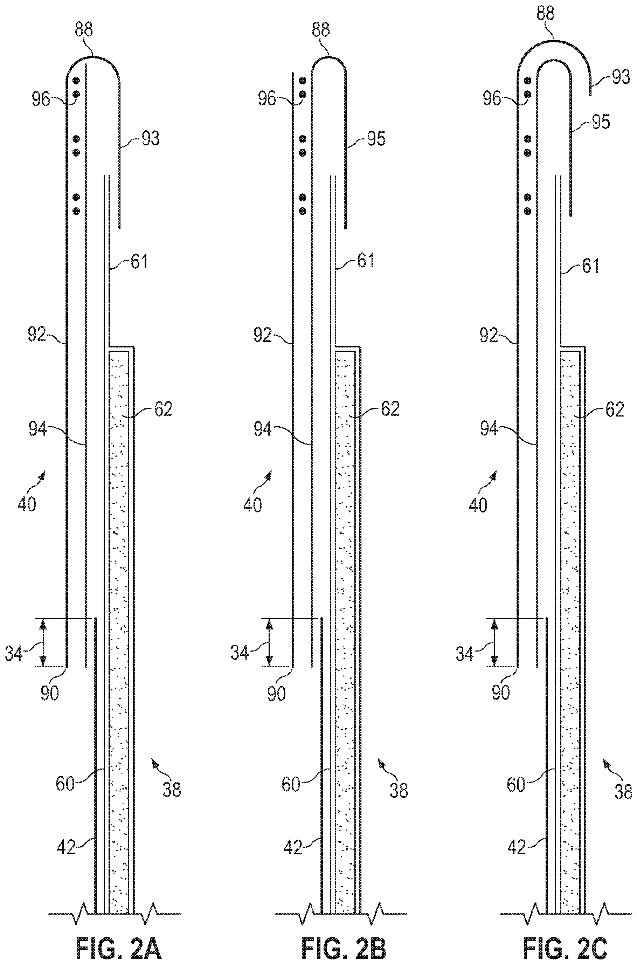

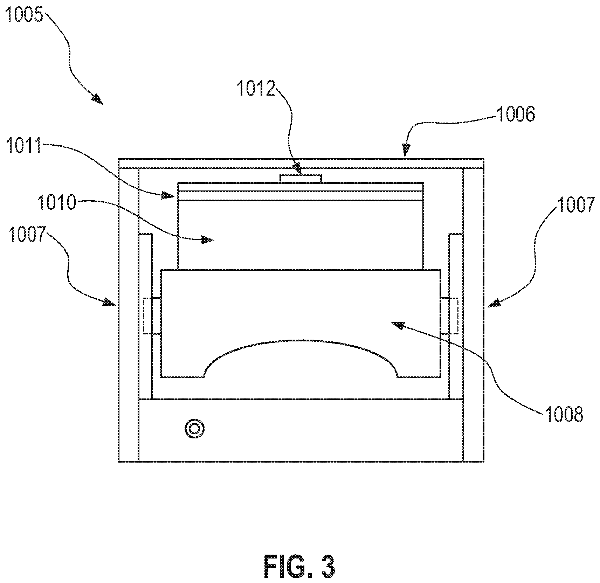

As exemplarily shown in Figures 2A and 3A-3C, the central chassis 38 may comprise a backsheet 60 and an outer cover layer 42 for covering the outer side of the backsheet 60. The backsheet 60 may be a water impermeable film. At least a portion of or the entirety of the outer cover layer 42 may be the elastic laminate of the present invention. The central chassis 38 may contain an absorbent core 62 for absorbing and containing body exudates disposed on the central chassis 38. As exemplified in in Figure 1A, the central chassis 38 may have a generally rectangular shape, left and right longitudinally extending side edges 48 (hereinafter may be referred to as "side edge") and front and back transversely extending end edges 50 (hereinafter may be referred to as "end edge"). The central chassis 38 also has a front waist panel 52 positioned in the front region 26 of the wearable article, a back waist panel 54 positioned in the back region 28, and a crotch panel 56 between the front and back waist panels 52, 54 in the crotch region 30. The center of the front belt 84 is joined to a front waist panel 52 of the central chassis 38, the center of the back belt 86 is joined to a back waist panel 54 of the central chassis 38, the front and back belts 84, 86 each having a left side panel and a right side panel 82 where the central chassis 38 does not overlap. The central chassis 38 may comprise one or more leg cuffs per side for gasketing the leg opening. At least a portion of, or at least one of, or all of, the leg cuffs may be the elastic laminate of the present invention.

While not depicted, the wearable article of the present invention may be a taped diaper having a longitudinal axis, a transverse axis, a body facing surface, and a garment facing surface. The wearable article may have a central chassis comprising a front region, a back region, and a crotch region, each defined by a laterally extending line notionally divided along the longitudinal axis in 3 equal lengths. The front region and/or the back region may be provided with fastening members for fastening the article to configure the waist opening and leg openings. The waist opening may comprise a waistband. The fastening member may be made by a connecting part connecting to the central chassis, a stretchable side panel which is stretchable in the lateral direction, and an engaging part having engaging elements such as hooks. The front region and/or the back region may be provided with a landing zone for receiving the engaging elements of the fastening member. The landing zone may be loops engageable with the hooks. At least a portion of, or the entirety of, the waistband, side panels, or landing zones of the wearable article may be the elastic laminate of the present invention.

The ring-like elastic belt 40 of the pant of the present invention acts to dynamically create fitment forces and to distribute the forces dynamically generated during wear. The proximal edge 90 is located closer than the distal edge 88 relative to the crotch panel 56 of the central chassis 38. The front and back belts 84, 86 may be joined with each other only at the side edges 89 at the seams to form a wearable article having a waist opening and two leg openings. Each leg opening may be provided with elasticity around the perimeter of the leg opening. For the belt-type pant, the elasticity around the leg opening may be provided by the combination of elasticity from the front belt 84, the back belt 86, and any from the central chassis 38.

The front belt 84 and back belt 86 of the pant are configured to impart elasticity to the belt 40. The front belt 84 and the back belt 86 may each be formed by the present elastic laminate comprising a plurality of elastic bodies 96 running in the transverse direction, an inner sheet 94 formed by the inner layer, and an outer sheet 92 formed by the outer layer. Optionally an outer sheet fold over 93 which is an extension of the outer sheet material may be formed by folding the outer sheet material, and/or an inner sheet fold over 95 which is an extension of the inner sheet material may be formed by folding the inner sheet material. The outer sheet 92 may be made of the same nonwoven substrate of the present invention as the outer cover layer 42 to provide integral aesthetic and tactile senses for the article.

When the central chassis 38 contains an absorbent core, some or all of the areas of the front or back belt 84, 86 overlapping the absorbent core may be made devoid of elasticity. Referring to Figure 1A, areas of the front waist panel 52 and back waist panel 54 in which the elastic bodies 96 are deactivated are shown in blank. For example, as seen in the back belt 86, the elastic bodies 96 overlapping the absorbent material non-existing region 61 and toward the distal edges of the absorbent core 62 may be disposed in active elasticity for good fit of the central chassis 38. This may be advantageous in preventing leakage.

Providing an inner or outer fold over 95, 93 is advantageous for avoiding the waist opening 88 ending in sharp edges of the front or back belt 84, 86. Further, any elastic bodies 96 in the front or back belt 84, 85 may be disposed at least about 2 mm away, or from about 5 mm to about 9 mm away from the waist opening, to avoid the waist opening to be sharp, and also to ensure that any elastic body is not accidentally exposed during manufacture or use. The inner or outer sheet fold over 95, 93 may extend toward the proximal edge such that there is overlap between the central chassis 38 by at least about 10 mm, or by at least about 15 mm, to secure integrity between the front and or back belt 84, 86 and central chassis 38.

Referring to Figure 2A, the front belt 84 and/or back belt 86 may comprise an outer sheet fold over 93 wherein the outer sheet fold over 93 is an extension of the outer sheet material, the outer sheet fold over 93 formed by folding the extended outer sheet material at the distal edge 88 of the belt. When the front belt 84 and/or back belt 86 is formed by the present elastic laminate, the outer layer is elongated beyond the elastic laminate, and folded over the elastic laminate such that at least a portion of the elastic belt comprises 1 layer of the inner layer sandwiched with 2 layers of the outer layer. When such structure is provided, the body-facing side of the outer sheet fold over region may be treated to have equal or higher hydrophobicity than the inner sheet, such that the hydrophilicity gradient of the layers of the elastic laminate is maintained.

Referring to Figure 2B, the front belt 84 and/or back belt 86 may comprise an inner sheet fold over 95 wherein the inner sheet fold over 95 is an extension of the inner sheet material, the inner sheet fold over 95 formed by folding the inner sheet material at the distal edge 88 of the belt. When the front belt 84 and/or back belt 86 is formed by the present elastic laminate, the inner layer is folded over itself such that at least a portion of the elastic belt comprises 2 layers of the inner layer. When such structure is provided, the body-facing side of the inner sheet fold over region may optionally be treated to have higher hydrophobicity than the inner sheet, such that the hydrophilicity gradient of the layers of the elastic laminate is maintained. For such configuration, the outer sheet 92 may not extend up to the waist opening created by the inner sheet 94. In this case, any elastic bodies may be disposed at least about 2mm away, or from about 5mm to about 9mm away from the distal edge of the outer sheet 92 to ensure that any elastic body is not accidentally exposed during manufacture or use.

Referring to Figure 2C, the front belt 84 and/or back belt 86 may comprise both an outer sheet fold over 93 and an inner sheet fold over 95. When the front belt 84 and/or back belt 86 is formed by the present elastic laminate, the inner layer is folded over itself such that at least a portion of the elastic belt comprises 2 layers of the inner layer, and the outer layer is further folded over the inner layer fold over. The inner layer fold over region extends proximally beyond the outer layer fold over region. When such structure is provided, the hydrophilicity gradient of the layers of the elastic laminate is maintained for at least the region wherein the inner sheet fold over is the body-facing surface. Meanwhile, the waist opening may be devoid of sharp edges, and the vicinity of the waist opening may be made lofty and undergarment like by having 4 layers of material. The outer sheet fold over region 93 may be from about 2 mm to about 15 mm, or from about 5 to about 10 mm. The inner sheet fold over 95 may extend further proximally to overlap the central chassis 38.

Referring to Figures 3A, 3B and 3C, all of, or at least a majority of the area of the front or back elastic belt between the waist opening 88 and the central chassis 38 of the present invention may be made of 3 layers comprising the inner layer and the outer layer, and an additional inner layer or an additional outer layer. Thus, the combined basis weight of the area of the front or back elastic belt between the waist opening 88 and the central chassis 38 may be kept low. Providing this area of low basis weight is advantageous in maintaining breathability of the overall elastic belt, which further contributes to sweat management. The combined basis weight of the area of the front or back elastic belt between the waist opening 88 and the central chassis 38 of the present invention may be less than about 100 g/m$^2$, or less than about 85 g/m$^2$, or less than about 75 g/m$^2$, or less than about 65 g/m$^2$.

Referring to Figure 1A, the transverse width LW of the back belt 86 in the uncontracted condition may be the same as the transverse width of the front belt 84 of the same condition. Such an article may be economically made. The longitudinal length LB of the back belt 86 between the back distal edge 88 and the back proximal edge 90 along its entire width LW of the back belt 86 may be approximately the same as the longitudinal length LF of the front belt 84 between the front distal edge 88 and the front proximal edge 90. In such configuration, the seams close the front and back belt 84, 86 side edges 89 of the same length for forming the article. Such an article may be economically made. The back belt 86 may have a greater longitudinal length LB between the back distal edge 88 and the back proximal edge 90 along its entire width LW of the back belt 86 in the transverse direction than the longitudinal length LF of the front belt 84 between the front distal edge 88 and the front proximal edge 90. In such configuration, when the wearable article is assembled to form the waist opening and the leg openings, the wearable article is folded along the transverse centerline T1 such that the front distal edge 88 is aligned with the back distal edge 88. The front side edge 89 is also aligned with a portion of the back side edge 89. Then the front belt 84 and the back belt 86 are joined at the front and back side edges 89 at the seams. The front and back proximal edges 90, however, may not be aligned to one another. The back proximal edge 90 may be disposed longitudinally closer than the front proximal edge 90 relative to the transverse center line T1 such that the proximal portion of the back side panel 82 extends toward the crotch panel 56 of the central chassis 38 beyond the front proximal edge 90. The side edge of the proximal portion of the back side panel 82 may not be joined to anywhere and free from attachment. Thus, the proximal portion of the back side panel 82 provides a buttock cover.

Referring to Figure 1A and 2A-2C, the front and back belts 84, 86 may be discontinuous with one another in the crotch region 30, such that the outer cover layer 42 is the garment-facing surface in the crotch region 30. The outer cover layer 42 may extend only partly in the longitudinal direction of the front waist panel 52 and the back waist panel 54 to leave the distal parts of the front waist panel 52 and the back waist panel 54 free of the outer cover layer 42. Namely, the longitudinal length of the outer cover layer 42 may be longer than the longitudinal length of the crotch panel 56 and shorter than the longitudinal length of the backsheet 60. By such configuration, the distal parts of the front waist panel 52 and the back waist panel 54 are devoid of the outer cover layer 42, providing better breathability and sweat management for the elastic belt 40. Further, this may provide cost saving of the outer cover layer 42 material. Accordingly, looking at the layers of elements between the garment facing surface and the backsheet 60 of the center chassis 38, there exists a transitional region 34 disposed on the front and back waist panel 52, 54 where the outer cover layer 42 is present. The longitudinal length of the transitional region 34 may be made as short as possible, for example, less than about 20 mm, or less than about 15 mm, or less than about 10 mm. Further, adhesive may be applied on the entire area of the transitional region 34, or the entire area leaving no more than up to 5 mm, in the longitudinal direction, from the distal edge of the transitional region 34. For providing attractive artwork for a wearable article in an economical manner, printing may be provided on the garment facing side of the backsheet 60. By providing the transitional region 34 as short as possible, applying adhesive to the transitional region 34 to enhance transparency, or simply avoiding displaying artwork in the transitional region 34, compromised appearance of the artwork over different layers of material between the artwork and the observer may be avoided. Referring to Figure 1A, artwork on the backsheet 60 may be printed in regions 40F and/or 40B.

The articles of the present invention provide improved sweat management properties, are easy to apply and comfortable to wear, while being economic to make.

**Test methods**

1. NMR MOUSE method

The NMR-MOUSE (Mobile Universal Surface Explorer) is a portable open NMR sensor equipped with a permanent magnet geometry that generates a highly uniform gradient perpendicular to the scanner surface (shown in Figure 3). A frame 1007 with horizontal plane 1006, made of glass-fiber reinforced plastic, supports the specimen and remains stationary during the test. A flat sensitive volume of the specimen is excited and detected by a surface rf coil 1012 placed on top of the magnet 1010 at a position that defines the maximum penetration depth into the specimen. By repositioning the sensitive slice across the specimen by means of a high precision lift 1008, the scanner can produce one-dimensional profiles of the specimen's structure with high spatial resolution.

An exemplary instrument is the Profile NMR-MOUSE model PM25 with High-Precision Lift available from Magritek Inc., San Diego, CA. Requirements for the NMR-MOUSE are a 50 $\mu$m resolution in the z-direction, a measuring frequency of 13.5 MHz, a maximum measuring depth of 25 mm, a static gradient of 8 T/m, and a sensitive volume (x-y dimension) of 40 by 40 mm$^2$. Before the instrument can be used, perform phasing adjustment, check resonance frequency and check external noise level as per the manufacturer's instruction. All measurements are conducted in a room controlled at 23°C $\pm$ 1°C and 50% $\pm$ 2% relative humidity.

The test solution is prepared: 0.9% w/v saline solution prepared as 9.0 g of NaCl diluted to 1 L deionized water. 2 mM/L of Diethylenetriaminepentaacetic acid gadolinium (III) dihydrogen salt (available from Sigma Aldrich) is added. After addition the solutions are stirred using a shaker at 160 rpm for one hour. Afterwards the solutions are checked to assure no visible undissolved crystals remain. The solution is prepared 10 hours prior to use.

Products for testing are conditioned at 23°C $\pm$ 1°C and 50% $\pm$ 2% relative humidity for two hours prior to testing.

Identify the elastic laminate of the wearable article and cut an 80.0 mm by 80.0 mm specimen from the elastic laminate. Ensure that only elastic laminate is cut out to enable a flat specimen 1022 on the frame. If the elastic laminate is attached to other components of the wearable article and a specimen cannot be cut without separating the elastic laminate, the elastic laminate should be carefully separated by appropriate techniques from those other components, e.g. by applying "Quik-Freeze®" type cold spray, or other suitable methods that do not permanently alter the properties of the elastic laminate.

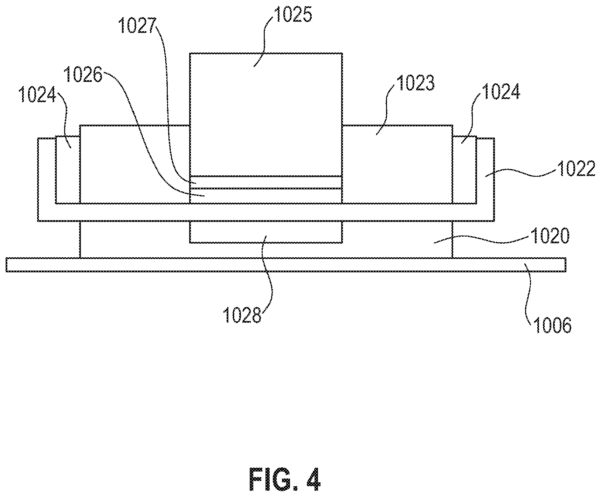

As illustrated in Fig. 4, the specimen 1022 is mounted on an 80 mm $\times$ 80 mm $\times$ height of 20 mm frame 1023 (different from the NMR MOUSE frame 1007 mentioned above), made of polycarbonate Makrolon®, with the second surface of the outer layer facing upwards using two pieces of double-sided tape 1024 on each edge to stretch the elastic laminate until the material is flat. The frame has an opening area of 40 mm $\times$ 40 mm into which a top marker is inserted. The top marker consists of a block 1025, made of polycarbonate Makrolon®, and a glass plate 1026 which is mounted to the block 1025 with double-sided tape 1027 (glass slide 1026 and tape 1027 are shown in exaggerated dimension in Fig. 4). The block 1025 has a dimension of 40 mm $\times$ 40 mm corresponding to the size of the opening area in the frame 1023. The high of the polymeric block is 147 mm. The glass plate 1026 has a thickness of 400 $\mu$m. The double-sided tape 1027 must be suitable to provide NMR Amplitude signal.

When the specimen 1022 is mounted on the frame 1023, the first surface of the inner layer is facing towards the NMR MOUSE instrument. Ensure the 40 mm $\times$ 40 mm opening area of the frame 1023, into which the top marker is inserted is covered by the specimen 1022.

Like the frame 1023, the sample holder 1020 has an opening 1028 of 40 mm $\times$ 40 mm. The depth of the opening in the sample holder 1028 is 400 $\mu$m. The sample holder has a dimension of 80 mm $\times$ 80 mm and a height of 1.4 mm (including the 400 $\mu$m depth). Place the sample holder 1020 in the middle of the NMR MOUSE (above rf coil 1012) on plane 1006 to ensure that the sensitive NMR volume is within the 40 mm $\times$ 40 mm opening 1028 where the liquid will be applied. Place the frame 1023 centered on top of the sample holder 1020, such that the top marker and opening 1028 are congruent and place the top marker onto the specimen 1022, such that glass plate 1026 is in contact with specimen 1022, to apply 0.25 psi on 40 mm $\times$ 40 mm. The top marker is used to define the dimension of the specimen by determining the surface of the sample holder 1020 and the specimen 1022 in the 40 mm $\times$ 40 mm opening area 1028.

To measure the specimen first 1-D Dry Distribution Profiles with and without the specimen 1022 are collected. Ensure that the prepared specimen on the instrument is aligned over top the rf coil 1012. Program the NMR-MOUSE for a Carr-Purcell-Meiboom-Gill (CPMG) pulse sequence consisting of a 90°x -pulse follow by a refocusing pulse of 180 °y -pulse using the following conditions:

Repetition Time = 500 ms
Number of Scans = 8
Number of Echoes = 8
Resolution = 50 $\mu$m

Step Size = -50 μm
Pulse Length = 5 μs
Echo Time = 90 μs
Echo Shift = 1 μs

Rx Phase of the NMR signal is optimized during the phase adjustment as described by the vendor to maximize the real part of the NMR signal which is used for data processing. A value of 230° was applied for our experiments. However, the optimal value may differ depending on the NMR instrument used, and hence the Rx phase should be optimized as described by the vendor. Pulse length for a 90° pulse depends on measurement depth which here is 5 mm and was determined to be 5μs based on the optimization procedure described by the vendor. If necessary, the depth can be adjusted using a spacer 1011 (see Fig. 3).

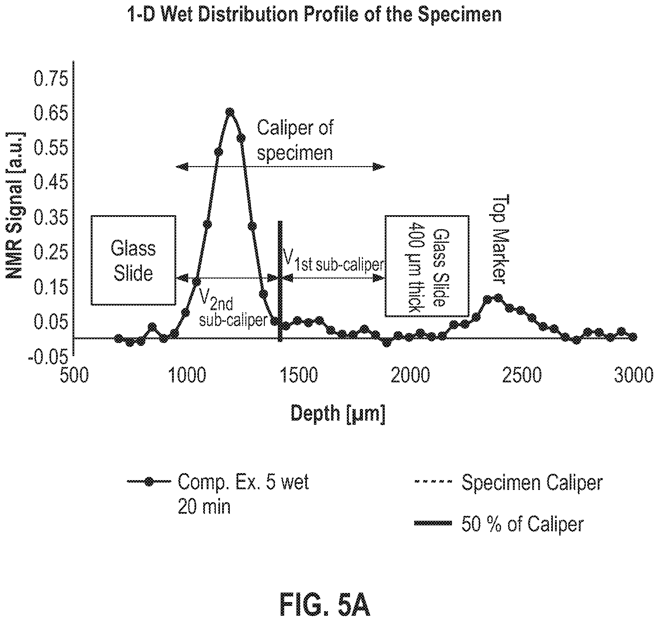

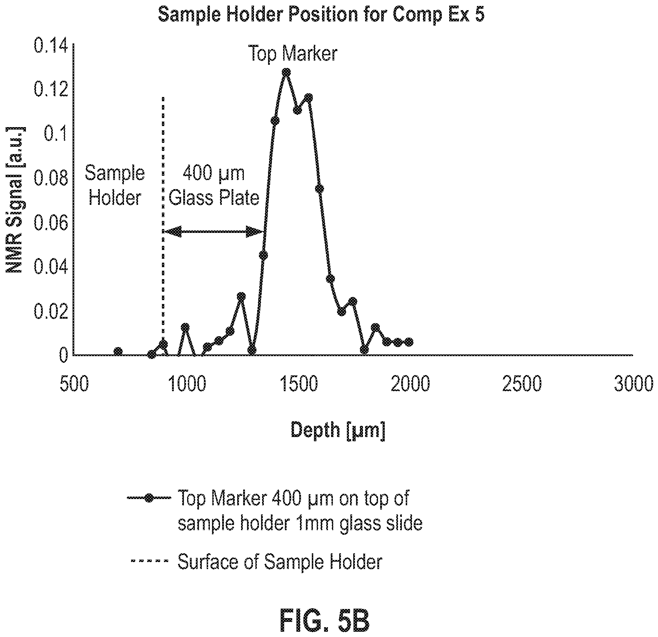

Collect NMR Amplitude data (in arbitrary units, a.u.) versus depth (μm) as the high precision lift steps through the specimen's depth. A representative graph with the specimen is shown in Fig. 5A and without the specimen is shown in Fig. 5B.

After the Dry Distribution Profile has been measured remove the top marker and the frame 1023 with the specimen 1022 attached thereto. Apply 400 μl of test solution (see above for preparation of test solution) in the opening 1028 of the sample holder 1020. Immediately afterwards place the frame 1023 with the specimen 1022 still attached thereto on top of the sample holder 1020 and insert the top marker into the frame such that glass plate 1026 is in contact with the specimen 1022, analogous to the procedure for taking the Dry Distribution Profile. Measure the NMR 1-D Wet Distribution Profile in the wet state of the specimen 1 minute after the specimen 1022 has been brought into contact with the test solution and the top marker has been added to the sample. This profile is used to calculate the Liquid Ratio of the first sub-caliper to the second sub-caliper value after one minute.

The NMR 1-D Wet Distribution Profile used to calculate Liquid Ratio of the first sub-caliper to the second sub-caliper after 10 minutes, and after 20 minutes is determined in the following way: The top marker is removed to allow the liquid to evaporate from the sample holder through the specimen for first 10 min. Evaporation step is carried out with no additional air ventilation, i.e. evaporation is not accelerated by air ventilation. The frame with the specimen remains on top of the sample holder during the complete evaporation time. After 10 min insert the top marker into the frame and place it again on top of the sample holder and -without further waiting- measure the NMR 1-D Wet Distribution Profile. This profile is used to calculate the Liquid Ratio of the first sub-caliper to the second sub-caliper value after ten minutes.

Repeat the procedure to allow another 10 min evaporation of liquid through the specimen 1022 (like for the first 10 minutes, this evaporation step is carried out with no additional air ventilation, i.e. evaporation is not accelerated by air ventilation), and re-assemble the top marker onto the sample holder, then measure the NMR 1-D Wet Distribution Profile in the wet state of the specimen 20 minutes. This profile is used to calculate the Liquid Ratio of the first sub-caliper to the second sub-caliper value after twenty minutes.

When taking off the top marker for the evaporation steps as described above, no further test solution is filled in to the opening 1028, i.e. test solution is only filled in once, after the Dry Distribution Profile has been taken.

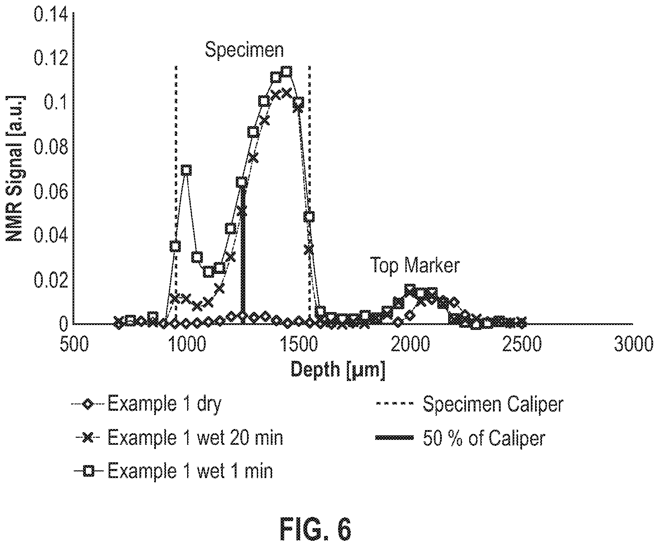

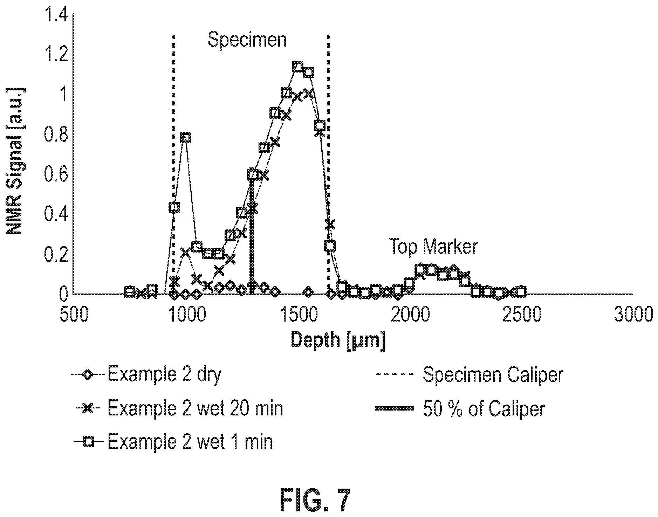

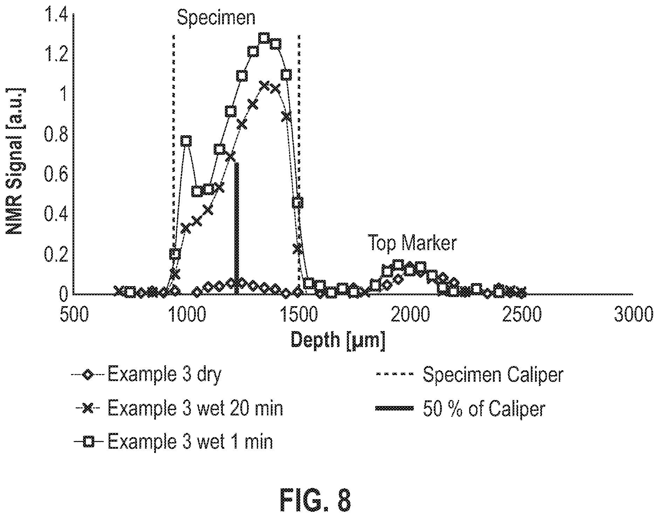

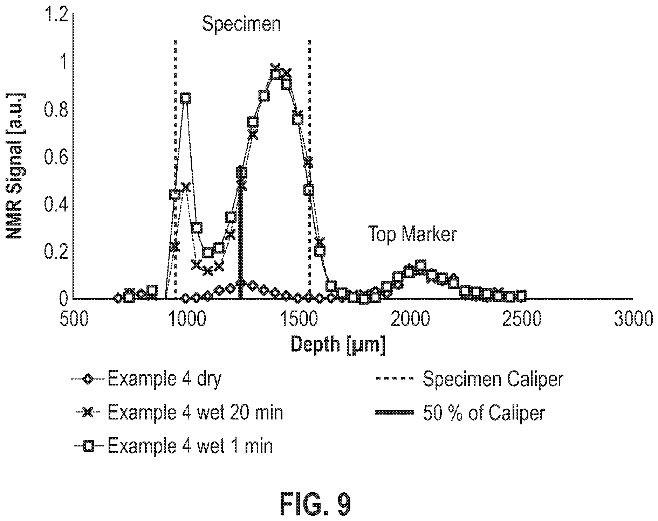

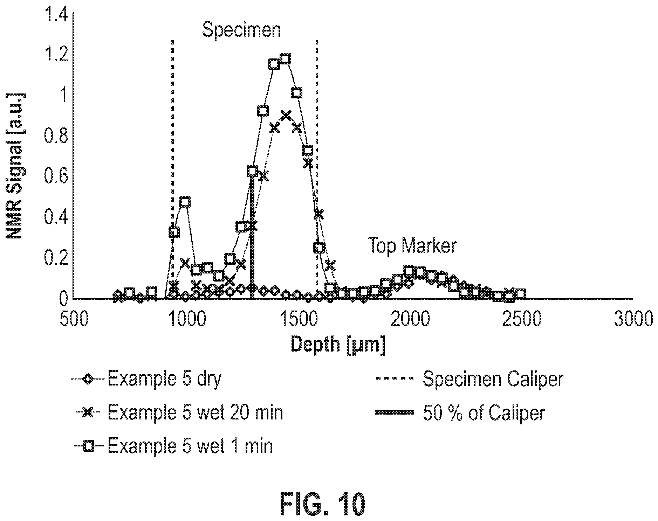

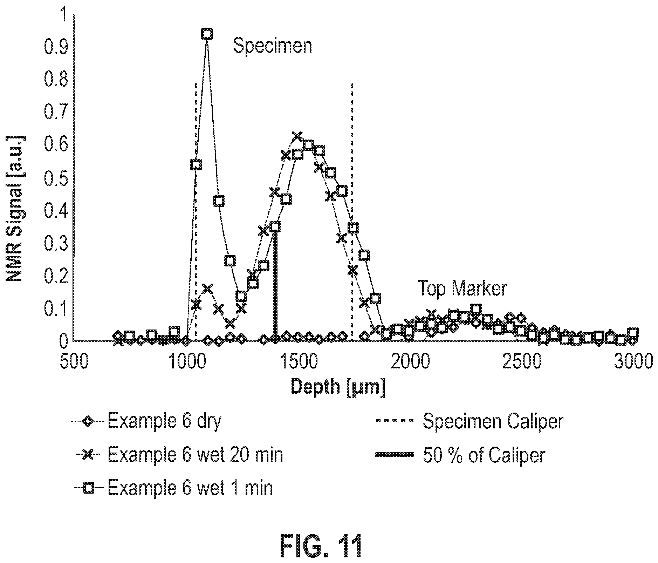

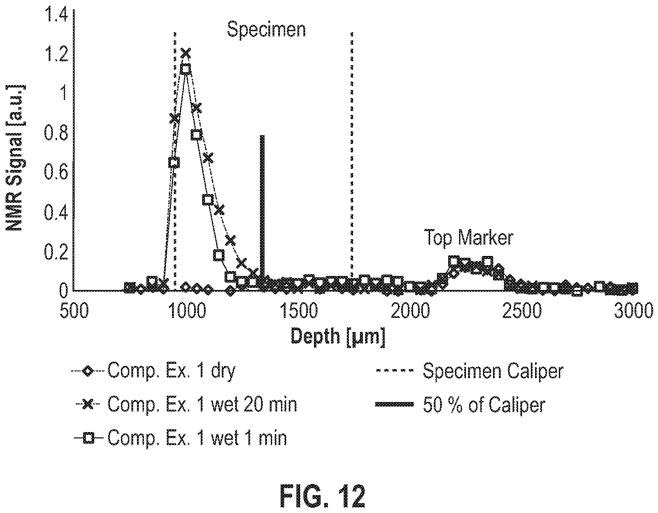

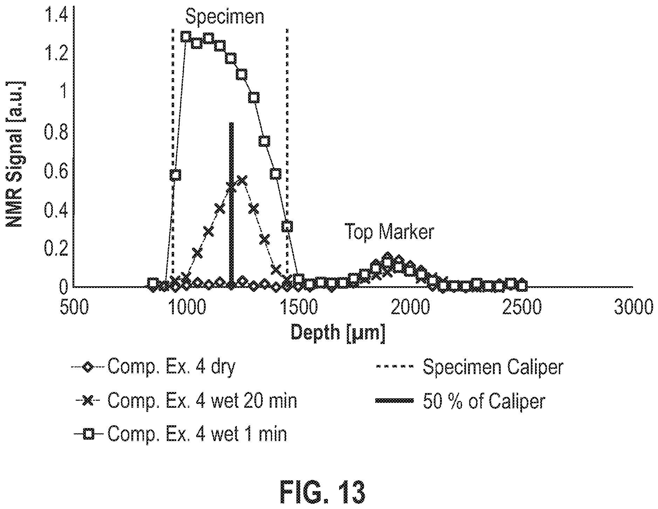

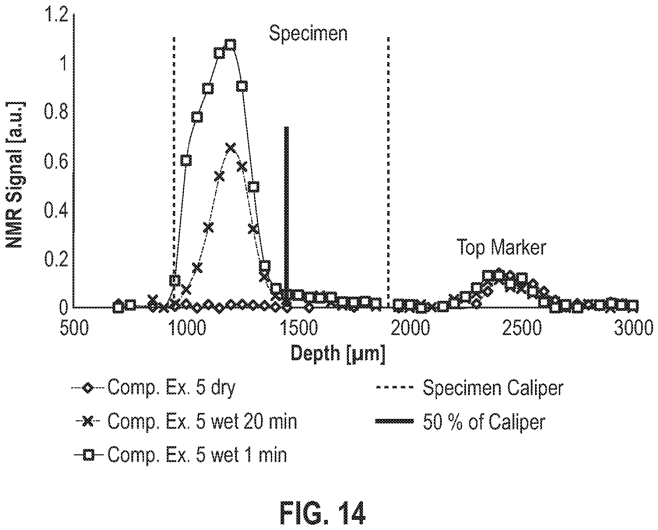

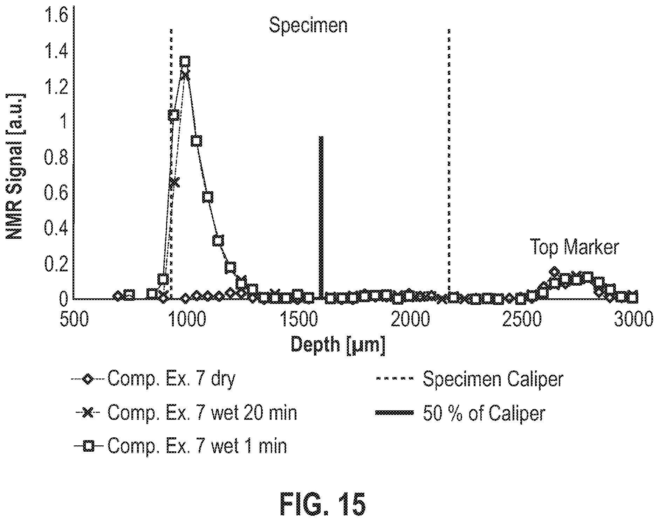

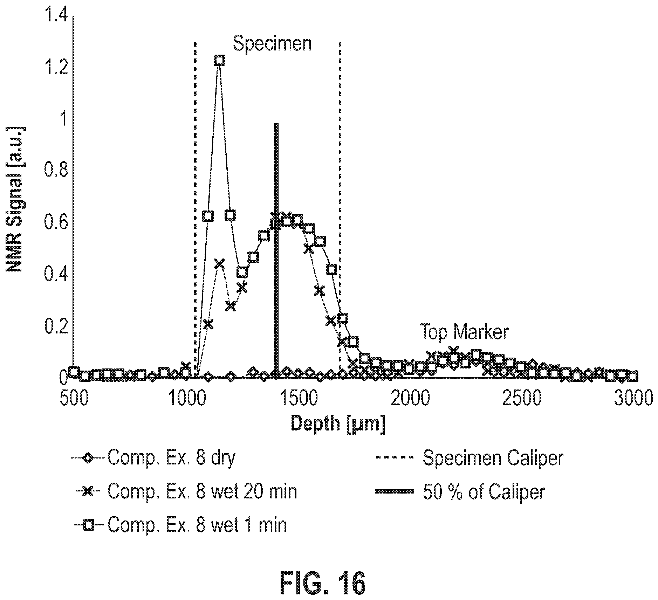

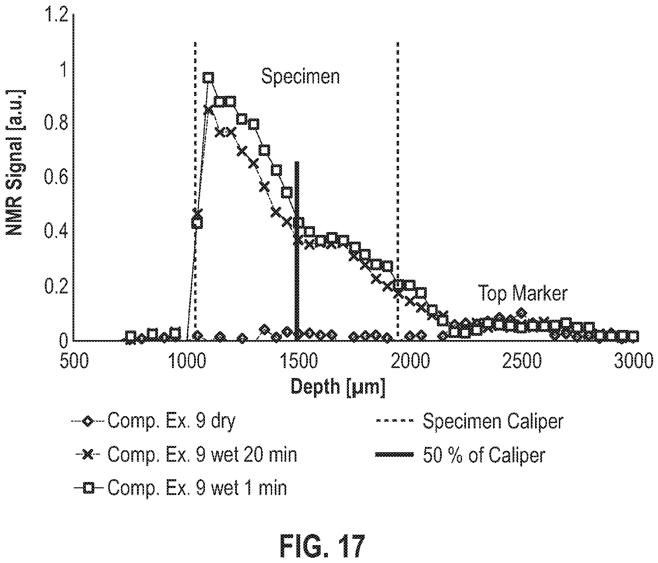

Figs. 6 to 17 show the Dry Distribution Profiles, as well as the Wet Distribution Profiles after 1 minute and 20 minutes of the examples and some of the comparative examples described below.

The liquid ratio of the first sub-caliper to the second sub-caliper is calculated as described below. Figure 5A shows a typical example for the liquid distribution as a function of depth.

Area calculation is made for any range of caliper of interest such as the total caliper in the specimen (i.e. the total caliper of the elastic laminate), the first sub-caliper of the specimen (i.e. the first sub-caliper of the elastic laminate), and the second sub-caliper of the specimen (i.e. the second sub-caliper of the elastic laminate):

$$Area_{Wet,Dry} = \sum_{i=1}^{n} \frac{X_{i+1} - X_i}{2} \times (S_{i+1} + S_i)$$

Where x is the depth in μm and S is the NMR Signal, n is the number of the data points.

Calculate the volume for the first sub-caliper and the second sub-caliper as can be seen below.

$$Volume_{first\ sub-caliper}\ [\mu l] = \frac{Area_{wet,first\ sub-caliper} - Area_{dry,first\ sub-caliper}}{Area_{wet,total\ caliper} - Area_{dry,total\ caliper}} \times 400\ [\mu l]$$

$$Volume_{second\ sub-caliper}\ [\mu l] = \frac{Area_{wet,second\ sub-caliper} - Area_{dry,second\ sub-caliper}}{Area_{wet,total\ caliper} - Area_{dry,total\ caliper}} \times 400\ [\mu l]$$

Calculate liquid ratio by dividing the volume in the first sub-caliper by the volume of the second sub-layer as can be seen below.

$$Liquid\ Ratio = \frac{Volume_{first\ sub-caliper}\ [\mu l]}{Volume_{second\ sub-caliper}\ [\mu l]}$$

Where Volume$_{first-sub-caliper}$ is the volume in 50 % of the caliper of the specimen (i.e. of the elastic laminate) and Volume$_{second-sub-caliper}$ is the volume in the remaining 50 % of the caliper of the flattened specimen on the frame as shown in Fig. 5A. The total caliper of the flattened out elastic laminate under 0.25 psi is determined by using the position of the top marker on top of the sample holder without any specimen shown in Fig. 5B and the position of the top marker out of the 1 D Wet Distribution Profile, see Fig. 5A, where total caliper of the elastic laminate is "caliper of specimen".

Each measurement (Dry Distribution Profile, Wet Distribution Profile after 1 minute, Wet Distribution Profile after 10 minutes, and Wet Distribution Profile after 20 minutes) is taken on one specimen only. Corresponding to each Wet Distribution Profile measured, a Liquid Ratio is calculated and reported as a dimensionless ratio to the nearest hundredth.

2. Preparation of Specimen for test methods on Effective Opening Area and Opening Rate; Relative Opening Rate; Fiber Diameter; Porosity and Void Cell Diameter; and Mean Flow Pore Size

When a nonwoven specimen is obtained from a finished product sample or laminate sample rather than obtained as a fresh material, the following procedures are taken.

A. To obtain a specimen, when available, an area free of deformation or wrinkling is selected. For the inner sheet 94 or outer sheet 92 of a wearable article, when available, area where the elasticity is deactivated is selected. The outer layer and inner layer are separated from the other components such as belt laminated nonwoven layers, or backsheet film by techniques such as applying "Quik-Freeze®" type cold spray, or other suitable methods that do not permanently alter the properties of the nonwoven composition. The technical face-side is the surface intended to be used as the garment-facing surface for the outer layer, and the body-facing surface for the inner layer. Care should be taken to prevent stretching of the nonwoven composition during the separation process. An 80mm by 80mm square shape is cut out using a cutter and a 64 cm$^2$ die for obtaining the specimen.

B. Any remaining adhesive is removed from the specimen by the following steps using Tetrahydrofuran (THF) as solvent.

1) In a hood, transfer 1 liter of THF into the 3 - 4 liter beaker.
2) Submerge specimen in the 1 liter of THF.
3) Place beaker on shaking table and stir gently for 15 minutes and keep solution with sample sit for 5 additional minutes.
4) Take specimen out of THF solution, and carefully squeeze THF solution out of specimen.
5) Let specimen air dry in hood for a minimum of 15 minutes.

Specimen are obtained from five (5) finished articles or laminates and cut out from the same area of each article or laminate, for each set of measurement. Specimen are pre-conditioned in a room maintained at 23 ± 2°C and 50 ± 5 % relative humidity, for at least 2 hours prior to testing.

3. Effective Opening Area and Opening Rate

Effective Opening Area and Opening Rate measurements are obtained by analyzing images of either an outer layer or an inner layer specimen independently, or both overlaying each other. Specimen images are generated using an optical microscope such as a Keyence 3D Measurement System VR-3200, or equivalent. Image analysis is performed using ImageJ software (version 1.46 or above, National Institutes of Health, USA, or equivalent). The specimen image needs to be distance calibrated with an image of a ruler to determine the image resolution, i.e. 42.4 pixels per mm. The layer specimen is backed with a black material prior to acquiring the image. A total of five replicate specimens are prepared for analysis.

Following the instrument manufacturer's recommended procedures, auto-focus the microscope and acquire a specimen image with a field of view size of 50 mm by 50 mm at a resolution of approximately 42.4 pixels per mm. In like fashion acquire images of the remaining four replicates.

Open a specimen image in ImageJ. Set the scale according to the resolution of the calibrated ruler image. Convert the image type to 8 bit. Using the minimum auto threshold, the 8-bit grayscale image is then converted to a binary image (with "zero" or "black" corresponding to the aperture regions) in the following way: If the histogram of gray level (GL) values (ranging from 0 to 255, one bin with propensity $P_i$ per gray level i) has exactly two local maxima, the threshold gray level value t is defined as that value for which Pt-1 > Pt and Pt ≤ Pt+1. If the histogram has greater than two local maxima, the histogram is iteratively smoothed using a windowed arithmetic mean of size 3, and this smoothing is performed iteratively until exactly two local maxima exist. The threshold gray level value t is defined as that value for which $P_{t-1} > P_t$ and $P_t \leq P_{t+1}$. This procedure identifies the gray level (GL) value for the minimum population located between the dark pixel peak of the aperture holes (openings) and the lighter pixel peak of the specimen material.

Remove outliers before measurement by setting the opening exclusion limit to 10 pixels in radius for dark outliers and 2 pixels in radius for bright outliers. Select the Analyze Particles function. Set the analysis to exclude the partial edge openings and calculate the average opening area via dividing the total area of all included openings by the number of openings, and record as the Effective Opening Area to the nearest 0.1 mm$^2$. Add up all the opening area values from the image, including both full and partial openings, and divide the sum by the total area of the field of view of the image, and record as the Opening Rate (%) to the nearest 0.1 %. In like fashion analyze the remaining 4 specimen images. Calculate and report the average Effective Opening Area to the nearest 0. 1 mm$^2$ and the average Opening Rate to the nearest 0.1 % for the total 5 replicates.

4. Relative Opening Rate

Relative Opening Rate is the opening rate of the outer layer and the inner layer combined, compared to the opening rate of the outer layer. Before preparation of specimen, the relationship of openings from the outer layer and the inner layer as the elastic laminate are observed. If the inner layer has no openings, the Relative Opening Rate is determined as 0%. If the inner layer has openings and the openings from the outer layer and the inner layer appear to completely or substantially match, the specimen is arranged as A). If the inner layer has openings and the openings from the outer layer and the inner layer appear to partially match or not match, the specimen is arranged as B).

A) The elastic laminate is treated according to the Preparation of specimen above to obtain the inner and outer layers. The inner and outer layers are overlayed such that the openings match each other as much as possible. The overlayed specimen is sent for measurement.

B) The elastic laminate is treated according to the Preparation of specimen above to obtain the inner and outer layers. The inner and outer layers are overlayed in 2 different degrees of overlap of openings, one which has the openings matched as much as possible, and another which has the openings unmatched as much as possible. The 2 types of overlayed specimen are sent for measurement, respectively. The average Relative Opening Rate of the 2 types of overlayed specimen is obtained.

The overlayed specimen are measured in the same manner as specified under "2. Effective Opening Area and Opening Rate" to obtain the Opening Rate of the overlayed specimen.

The Relative Opening Rate is obtained as such. When there is a complete match between the inner layer and the outer layer, the Relative Opening Rate is 100%.

$$\text{Relative Opening Rate } (\%) = \text{Opening Rate of overlayed specimen} / \text{Opening Rate of outer layer} \times 100$$

5. Fiber Diameter

Fiber Diameter is obtained as such. Scanning Electron Microscope images are taken using Hitachi TM3000 Bench-top SEM running Hitachi 3D-viewer software, or equivalent instrument. The specimen is cut into 20 mm by 20 mm and submerged in liquid nitrogen and an edge is fractured with a razor blade (stainless steel coated, single edge industrial blades, 62-0165). Fractured specimen is adhered to SEM mounts using double-sided tape (e.g. copper, carbon). The specimen is sputter Au coated and are viewed in the SEM. The SEM images are acquired from top view, and x-section view. Fiber diameter and width measurements are made using the manual line tool in SEM operating software.

6. Porosity and Void Cell Diameter

Porosity and 3-dimensional void cell spacing is obtained by image analysis by first acquiring the image using X-ray Micro-CT.

An 8 mm × 8 mm punch is used to physically extract a representative region of specimen. The 8 mm diameter sample is then placed in a sample holder with an inner diameter of 10 mm. The sample holder is then placed in an X-ray scanner such as GE Phoenix v|tome|x m (GE Sensing & Inspection Technologies GmbH, Niels-Bohr-Str. 7, 31515 Wunstorf, Germany). The scanning parameters used are nano-tube; voltage: 40 kV; current: 400 $\mu$A; tube mode: 1; timing: 2000 ms; averaging: 2; skip frames: 1; number of images: 2000. The resulting data set is 2014 × 2014 × 2014 voxels (volume pixels) with attenuation values represented as 16 bit integers. Each voxel has a diameter of 5 microns. For each type of specimen, three replicates are performed.

The 16-bit data was converted to 8-bit using a scaling factor of 0.01. A cylinder sub volume within the specimen of 7 mm in diameter and height around 90% in specimen height was cropped out from the original dataset for further calculation. To make measurements of Porosity and 3-dimenional void cell size distribution, the following steps were performed:

1) An automated thresholding algorithm, e.g. Otsu's method as a well-known thresholding method that is implemented in Matlab (Reference: Nobuyuki Otsu, "A Threshold Selection Method from Gray-Level Histograms", IEEE Transactions on Systems, Man, and Cybernetics, Vol. SMC-9, No. 1, January 1979) was applied to each of the datasets resulting in a binary image (0-1) representing the fibers (1) and void space (0).
2) The calculation of mean value of the resulting binary volume (0 - void space, 1 - fibers), then provides the % fibers in the region of interest. By subtracting this value from 100%, the porosity is calculated.
3) The void space is then fitted with spheres of different sizes, where larger spheres cover up smaller spheres. This final tessellation of the void space provides a distribution of spheres that completely cover the void space. The volume weighted mean diameter represents the mean void cell size. This is implemented through Matlab Code (Matlab R2016B, Natick, MA) as module in Avizo 9.2.0. This method is based on the paper published by Tor Hildebrand (Reference: T. Hildebrand and P. Ruegsegger, "A new method for the model-independent assessment of thickness in three-dimensional images", Journal of Microscopy, Vol. 185, 67-75, 1996).
4) The resulting measurements are brought into Excel spreadsheet. The values of the three replicates for each specimen are then averaged together to produce a single value for both porosity and void cell diameter.

7. Mean Flow Pore Size

Mean Flow Pore Size of nonwoven is characterized by the gas-liquid displacement method according to ASTM F316, using a capillary flow porometer such as Porolux™ 100 NW (Porometer N.V., Belgium). The porometry measurement follows the Young-Laplace equation, $P = 4 * \gamma * \cos(\theta) / D$, where D is the pore size diameter, P is the pressure measured, $\gamma$ is the surface tension of the wetting liquid, and $\theta$ is the contact angle of the wetting liquid with the sample. The procedures are the following:

1) Wet the specimen with a liquid of low surface tension and low vapor pressure, for example, a commercial wetting liquid Porefil (Prorometer N.V., Belgium) with surface tension of 16 mN/m. Consequently, all pores are filled with the liquid.
2) An inert gas is used to displace wetting liquid from pores and gas flow rate is normally measured using flow meters. The liquid is blown out of the specimen by gradually increasing the gas pressure. When further increasing the pressure, gas flows through small pores until all the pores are emptied. Record the gas pressure and gas flow rate when liquid is being expelled.
3) After the wet run, the measurement of the same sample in dry state is carried out.
4) The pore size parameters are calculated by comparing the pressure-flow rate curves from the wet run and dry run according to ASTM F316.

**EXAMPLES**

The following nonwoven layers 1 to 6 have been assembled into elastic laminates of Examples 1 to 7 and Comparative Examples 8 and 9:

Nonwoven 1: 15gsm spun calendar-bonded material made of hydrophobic PP monofilament, having fiber denier of 2dpf, supplied by Fibertex.

Nonwoven 2: 20gsm air-through carded material made of PE/PET bicomponent fibers having fiber denier of 2dpf, treated hydrophobically with tradename "R11", supplied by Xiamen Yanjan New Material Co. Ltd.

Nonwoven 3: 20gsm air-through carded material made of PE/PET bicomponent fibers having fiber denier of 2dpf, treated hydrophobically and apertured with tradename "J201-200", supplied by Xiamen Yanjan New Material Co. Ltd.

Nonwoven 4: 20gsm air-through carded material made of PE/PET bicomponent fibers having fiber denier of 2dpf, treated hydrophilically and apertured with tradename "DZ203-200", supplied by Xiamen Yanjan New Material Co. Ltd.

Nonwoven 5: 20gsm air-through carded material made of PE/PET bicomponent fibers having fiber denier of 2dpf, treated hydrophilically with tradename "Z08", supplied by Xiamen Yanjan New Material Co. Ltd.

Nonwoven 6: 13gsm SMS material comprising spunbond and meltblown layers made of hydrophobic PP monofilament with tradename "SM13011", supplied by First Quality Nonwovens, Inc.

Nonwovens 1 to 6 have been assembled into elastic laminate as follows:

**Table 1:** Materials of Examples 1 to 5 and Comparative Example 8

|  | Example 1 | Example 2 | Example 3 | Example 4 | Example 5 | Comparative Example 8 |
|---|---|---|---|---|---|---|
| Inner layer material | Nonwoven 2 No aperture | Nonwoven 3 Apertured | Nonwoven 1 No aperture | Nonwoven 2 No aperture | Nonwoven 3 Apertured | Nonwoven 6 No aperture |
| Outer layer material | Nonwoven 5 No aperture | Nonwoven 4 Apertured | Nonwoven 4 Apertured | Nonwoven 4 Apertured | Nonwoven 5 No Aperture | Nonwoven 5 No Aperture |
| Inner layer basis weight [g/m²] | 20 | 20 | 15 | 20 | 20 | 13 |
| Outer layer basis weight [g/m²] | 20 | 20 | 20 | 20 | 20 | 20 |
| Outer layer thickness [μm] | 284 | 632 | 632 | 632 | 284 | 284 |

Also, three elastic laminates have been made wherein the inner and/or outer layer were folded over:

Table 2: Materials of Examples 6 and Comparative Example 9

|  | Example 6 | Comparative Example 9 |
|---|---|---|
| Inner layer material | Nonwoven 1 No aperture | Nonwoven 1 No aperture |
| Outer layer material | Nonwoven 4 Apertured | Nonwoven 4 Apertured |
| Inner layer basis weight [g/m²] | 15 | 15 |

(continued)

|  | Example 6 | Comparative Example 9 |
|---|---|---|
| Outer layer basis weight [g/m$^2$] | 20 | 20 |
| Outer layer thickness [μm] | 632 | 632 |
|  | inner layer is elongated beyond the elastic laminate, and the elongated part of the inner layer is folded over itself (i.e. not folded over the outer layer), the folded over part of the inner layer thereby forming a third layer | outer layer is elongated beyond the elastic laminate, and the elongated part of the outer layer is folded over the elastic laminate (i.e. over the inner layer), the folded over part of the outer layer thereby forming a third layer wherein the third layer is not treated to have equal or higher hydrophobicity than the inner layer |

Liquid Ratio after 1 minute and after 20 minutes has been determined for Examples 1 to 6, as well as for Comparative Example 8, see Table 3 (Examples 1 to 6) and Table 4 (Comparative Examples 8 and 9).

Moreover, Liquid Ratio after 1 minute and after 20 minutes has been determined for the elastic belt (being formed by an elastic laminate) for the commercially available disposable absorbent pants (Comparative Examples 1 to 7) according to Table 4:

Table 3: Liquid Ratio for Examples 1 to 6

| Example No. | Liquid Ratio after 1 minute | Liquid Ratio after 10 minutes | Liquid Ratio after 20 minutes |
|---|---|---|---|
| 1 | 2.40 | 4.22 | 5.14 |
| 2 | 2.71 | 5.61 | 6.82 |
| 3 | 1.85 | 2.04 | 2.24 |
| 4 | 2.02 |  | 3.39 |
| 5 | 4.18 | 9.56 | 10.86 |
| 6 | 1.44 | 2.83 | 4.29 |

Table 4: Liquid Ratio for Comparative Examples 1 to 9

| Comparative Example No. | Description | Liquid Ratio after 1 minute | Liquid Ratio after 10 minutes | Liquid Ratio after 20 minutes |
|---|---|---|---|---|
| 1 | Goo.N Angel Pants; China, purchased September 2018 | 0.09 | 0.05 | 0.04 |
| 2 | Anerle Pants (Premium product); China, purchased 2016 | 0.02 | 0.03 | 0.03 |
| 3 | Moony Natural Taped Diaper; Japan, purchased April 2018 | 0.03 | 0.04 | 0.04 |
| 4 | Hasocare Adult Incontinence Pants; Japan, purchased July 2018 | 0.70 | 1.24 | 1.34 |
| 5 | Moony Sweat Pants with "sweat patch" (hydrophilic layer attached to the skin-facing surface of the inner belt layer (i.e. hydrophilic layer is in direct contact with skin during use); Japan, purchased June 2018 | 0.06 | 0.09 | 0.09 |

(continued)

| Comparative Example No. | Description | Liquid Ratio after 1 minute | Liquid Ratio after 10 minutes | Liquid Ratio after 20 minutes |
|---|---|---|---|---|
| 6 | JP Merries Pants; Japan, purchased 2018 | 0.11 | 0.18 | 0.23 |
| 7 | Kao Adult Slim Pants; Japan; purchased July 2018 | 0.00 | 0.00 | 0.01 |
| 8 | see Table 1 | 0.85 | 0.86 | 1.58 |
| 9 | See Table 2 | 0.55 | 0.56 | 0.57 |

For Examples 1 to 5, the following further parameters have been determined:

**Table 5:** Examples 1-5

| | Example 1 | Example 2 | Example 3 | Example 4 | Example 5 |
|---|---|---|---|---|---|
| Inner layer Opening Rate [%] Effective Opening Area [mm$^2$] | NA | 8.8/0.3 | NA | NA | 8.8/0.3 |
| Outer layer Opening Rate [%] Effective Opening Area [mm$^2$] | NA | 9.110.3 | 9.110.3 | 9.110.3 | NA |
| Relative Opening Rate [%)] | NA | 6.5% | 0 | 0 | 0 |
| Porosity comparison | inner and outer are same | inner and outer are same | Inner smaller | inner and outer are same | inner and outer are same |
| Void Cell Diameter comparison | inner and outer are same | inner and outer are same | Inner smaller | inner and outer are same | inner and outer are same |

**Claims**

1. A wearable article (20) comprising an elastic laminate, the elastic laminate comprising an inner nonwoven layer and an outer nonwoven layer, wherein the inner layer and the outer layer are directly joined with each other over an area of from about 5% to about 50% in that the inner layer and the outer layer are directly secured to each other by applying adhesive agents;

   the inner layer being closer to the body of the wearer than the outer layer when the article is worn, the inner layer having a first surface facing away from the outer layer and a second surface facing towards the outer layer, the outer layer having a higher hydrophilicity than the inner layer and having a first surface facing towards the inner layer and a second surface facing away from the inner layer, the inner layer being hydrophobic and the outer layer being hydrophilic;

   the laminate having a total caliper when subjected to the NMR MOUSE method as defined herein, the total caliper having a first sub-caliper corresponding to 50% of the total caliper starting from the outer layer's second surface and extending towards the inner layer's first surface, and a second sub-caliper corresponding to the remaining 50% of the total caliper starting from the inner layer's first surface and extending towards the outer layer's second surface, and

   wherein the elastic laminate has a Liquid Ratio of the first sub-caliper to the second sub-caliper of at least 1.1 after 1 minute according to the NMR MOUSE method set out herein;

   wherein, in the elastic laminate, the basis weight of the inner layer is not greater than the basis weight of the outer layer.

2. The wearable article (20) of claim 1, wherein the elastic laminate has a Liquid Ratio of the first sub-caliper to the second sub-caliper of at least 2.0 after 20 minutes according to the NMR MOUSE method set out herein.

3. The wearable article (20) of claim 1 or 2, wherein at least 40%, or at least 50% of the surface area of the elastic laminate, determined when the elastic laminate is stretched such that the inner and outer layer are flattened out, does not comprise any further layer except the inner and outer layer.

4. The wearable article (20) of any of the preceding claims, wherein the inner and outer layer are non-elastic.

5. The wearable article (20) of any of the preceding claims, wherein the inner layer is spunbond nonwoven, or a carded air-through bonded nonwoven.

6. The wearable article (20) of any of the preceding claims, wherein the outer layer is an air-through bonded carded nonwoven.

7. The wearable article (20) according to any of the preceding claims, wherein the elastic laminate further comprises a plurality of elastic bodies, such as elastic strands and/or elastic ribbons, provided between the inner and outer layer.

8. The wearable article (20) of claim 7, wherein the article has a longitudinal direction and a transverse direction, wherein, in the elastic laminate, the elastic bodies (96) extend along the transverse direction and are spaced apart from each other in the longitudinal direction of the article.

9. The wearable article (20) of claim 7 or 8, wherein the elastic bodies (96) and the inner and outer layer are at least partially joined by means of an adhesive.

10. The wearable article (20) according to Claim 7, 8 or 9, wherein the inner and outer layers and the plurality of elastic bodies (96) are at least partially joined by means of directly joining the fibers of the first layer and the second layer.

11. The wearable article (20) of any of the preceding claims, wherein the outer layer has a plurality of openings, such as apertures, in the second layer at an Opening Rate of from about 5% to about 50%, preferably from about 5% to about 30%, according to the test method set out herein.

12. The wearable article (20) of claim 11, wherein the outer layer has an Effective Opening Area of from 0.1 mm$^2$ to 25 mm$^2$, preferably from 0.1 mm$^2$ to 10 mm$^2$, according to the test method herein.

13. The wearable article (20) of any of the preceding claims wherein, in the elastic laminate, the inner layer has a plurality of openings, such as apertures, at an Opening Rate of from 5% to 30%, preferably from 5% to 15%, and the inner layer an Effective Opening Area of from 0.1 mm$^2$ to 25 mm$^2$, preferably from 0.1 mm$^2$ to 10 mm$^2$, according to the test method herein.

14. The wearable article (20) of any of the preceding claims wherein, in the elastic laminate, the porosity of the inner layer is smaller than the porosity of the outer layer, according to the test method herein.

15. The wearable article (20) of any of the preceding claims, wherein, in the elastic laminate, the inner layer has a basis weight of from 5 g/m$^2$ to 45 g/m$^2$, and the outer layer has a basis weight of from 10 g/m$^2$ to 45 g/m$^2$.

16. The wearable article (20) of any of the preceding claims, wherein the second layer has a thickness of at least 50 $\mu$m, preferably at least 80 $\mu$m according to the test method set out herein.

17. The wearable article (20) of any of the preceding claims, wherein the elastic laminate has an elongation rate of at least 110 % in at least one direction.

18. The wearable article (20) of any preceding claims, wherein the elastic laminate is used as a component selected from the group consisting of an elastic belt (40), a waistband, a side panel, leg cuffs, and an outer cover.

19. The wearable article (20) of any of the preceding claims, wherein the elastic laminate is comprised by, or forms an elastic belt (40) formed of a front belt (84) and a back belt (86).

20. The wearable article (20) of claim 18 or 19, wherein the elastic belt (84) comprises an outer layer fold over region wherein the outer layer is elongated beyond the elastic laminate, and the elongated part of the outer layer is folded over the elastic laminate, the folded over part of the outer layer thereby forming a third layer such that at least a portion of the elastic belt (84) comprises the inner layer sandwiched between the outer layer and the third layer, wherein the third layer facing towards the body of the wearer in use is treated to have equal or higher hydrophobicity than the inner layer.

21. The wearable article (20) of claim 18 or 19, wherein the elastic belt (84) comprises an inner layer fold over region wherein the inner layer is elongated beyond the elastic laminate, and the elongated part of the inner layer is folded over itself, the folded over part of the inner layer thereby forming a third layer such that at least a portion of the elastic belt (40) comprises the inner, outer and third layer, wherein the third layer facing towards the skin of the wearer in use is optionally treated to have higher hydrophobicity than the inner layer.

22. The wearable article (20) of claim 21, wherein the elastic belt (40) further comprises an outer layer fold over region wherein the outer layer is elongated beyond the elastic laminate and the elongated part of the outer layer is folded over the third layer, the folded over part of the outer layer thereby forming a fourth layer such that at least a portion of the elastic belt (40) comprises the inner layer and third layer sandwiched between outer layer and the fourth layer, wherein the third layer extends beyond the fourth layer, the extension of the fourth layer forming less than 20%, or less than 10% of the surface area of the elastic laminate, determined when the elastic laminate is stretched such that the inner and outer layer are flattened out.

23. The wearable article (20) of any of Claims 18-22 comprising a central chassis (38) and a ring-like elastic belt (40), the ring-like elastic belt (40) being formed of the elastic laminate and consisting of a front belt (84) and a back belt (86); the center of the front belt (84) being joined to a front waist panel (52) of the central chassis (38), the center of the back belt (86) being joined to a back waist panel (54) of the central chassis (38), and the remainder of the central chassis (38) forming a crotch region (30), the front and back belt (84, 86) each having a left side panel and a right side panel (82) where the central chassis (38) does not overlap, and the transverse edges of the front belt (84) and the back belt (86) being joined by a seam to form a waist opening (88) and two leg openings; wherein the front belt (84) and the back belt (86) are discontinuous of each other in the crotch region (30) in the longitudinal direction; the central chassis (38) comprising an outer cover layer (42) on the garment-facing surface and a backsheet (60) attached to the inner surface of the outer cover layer; wherein the longitudinal length of the outer cover layer (42) is longer than the longitudinal length of the crotch region (30) and shorter than the longitudinal length of the backsheet (60).

**Patentansprüche**

1. Tragbarer Artikel (20), umfassend ein elastisches Laminat, das elastische Laminat umfassend eine Innenvliesschicht und eine Außenvliesschicht, wobei die Innenschicht und die Außenschicht über einen Bereich von etwa 5 % bis etwa 50 % direkt miteinander verbunden sind, wobei die Innenschicht und die Außenschicht durch Aufbringen von Klebemitteln direkt aneinander befestigt sind;

wobei die Innenschicht näher an dem Körper des Trägers als die Außenschicht ist, wenn der Artikel getragen wird, wobei die Innenschicht eine erste Oberfläche, die von der Außenschicht abgewandt ist, und eine zweite Oberfläche, die der Außenschicht zugewandt ist, aufweist,
wobei die Außenschicht eine höhere Hydrophilie als die Innenschicht aufweist und eine erste Oberfläche, die der Innenschicht zugewandt ist, und eine zweite Oberfläche, die von der Innenschicht abgewandt ist, aufweist,
wobei die Innenschicht hydrophob ist, und die Außenschicht hydrophil ist;
wobei das Laminat eine Gesamtstärke aufweist, wenn sie dem NMR-MOUSE-Verfahren, wie hierin definiert, unterzogen wird, wobei die Gesamtstärke eine erste Unterstärke, die 50 % der Gesamtstärke entspricht, die von der zweiten Oberfläche der Außenschicht ausgeht und sich zu der ersten Oberfläche der Innenschicht erstreckt, und eine zweite Unterstärke, die den verbleibenden 50 % der Gesamtstärke entspricht, die von der ersten Oberfläche der Innenschicht ausgeht und sich zu der zweiten Oberfläche der Außenschicht erstreckt, aufweist, und
wobei das elastische Laminat ein Flüssigkeitsverhältnis der ersten Unterstärke zu der zweiten Unterstärke von wenigstens 1.1 nach 1 Minute gemäß dem hierin dargelegten NMR-MOUSE-Verfahren aufweist;
wobei, in dem elastischen Laminat, das Flächengewicht der Innenschicht nicht größer als das Flächengewicht der Außenschicht ist.

2. Tragbarer Artikel (20) nach Anspruch 1, wobei das elastische Laminat ein Flüssigkeitsverhältnis der ersten Unterstärke zu der zweiten Unterstärke von wenigstens 2.0 nach 20 Minuten gemäß dem hierin dargelegten NMR-MOUSE-Verfahren aufweist.

3. Tragbarer Artikel (20) nach Anspruch 1 oder 2, wobei wenigstens 40 % oder wenigstens 50 % des Oberflächenbereichs des elastischen Laminats, der bestimmt wird, wenn das elastische Laminat derart gedehnt ist, dass die Innen- und die Außenschicht abgeflacht sind, keine weitere Schicht, außer der Innen- und der Außenschicht, umfasst.

4. Tragbarer Artikel (20) nach einem der vorstehenden Ansprüche, wobei die Innen- und die Außenschicht nicht elastisch sind.

5. Tragbarer Artikel (20) nach einem der vorstehenden Ansprüche, wobei die Innenschicht ein Spinnvliesvlies oder ein kardiertes luftdurchleitungsgebundenes Vlies ist.

6. Tragbarer Artikel (20) nach einem der vorstehenden Ansprüche, wobei die Außenschicht ein luftdurchleitungsgebundenes kardiertes Vlies ist.

7. Tragbarer Artikel (20) nach einem der vorstehenden Ansprüche, wobei das elastische Laminat ferner eine Vielzahl von elastischen Körpern, wie elastische Faserbündel und/oder elastische Bänder, umfasst, die zwischen der Innen- und der Außenschicht bereitgestellt sind.

8. Tragbarer Artikel (20) nach Anspruch 7, wobei der Artikel eine Längsrichtung und eine Querrichtung aufweist, wobei, in dem elastischen Laminat, sich die elastischen Körper (96) entlang der Querrichtung erstrecken und in der Längsrichtung des Artikels voneinander beabstandet sind.

9. Tragbarer Artikel (20) nach Anspruch 7 oder 8, wobei die elastischen Körper (96) und die Innen- und die Außenschicht wenigstens teilweise mittels eines Klebers verbunden sind.

10. Tragbarer Artikel (20) nach Anspruch 7, 8 oder 9, wobei die Innen- und die Außenschicht und die Vielzahl von elastischen Körpern (96) wenigstens teilweise mittels direktem Verbinden der Fasern der ersten Schicht und der zweiten Schicht verbunden sind.

11. Tragbarer Artikel (20) nach einem der vorstehenden Ansprüche, wobei die Außenschicht eine Vielzahl von Öffnungen, wie Aperturen, in der zweiten Schicht bei einer Öffnungsrate von etwa 5 % bis etwa 50 %, vorzugsweise von etwa 5 % bis etwa 30 %, gemäß dem hierin dargelegten Prüfverfahren aufweist.

12. Tragbarer Artikel (20) nach Anspruch 11, wobei die Außenschicht eine wirksame Öffnungsfläche von 0,1 mm$^2$ bis 25 mm$^2$, vorzugsweise von 0,1 mm$^2$ bis 10 mm$^2$, gemäß dem Prüfverfahren hierin aufweist.

13. Tragbarer Artikel (20) nach einem der vorstehenden Ansprüche, wobei, in dem elastischen Laminat, die Innenschicht eine Vielzahl von Öffnungen, wie Aperturen, bei einer Öffnungsrate von 5 % bis 30 %, vorzugsweise von 5 % bis 15 %, und die Innenschicht eine wirksame Öffnungsfläche von 0,1 mm$^2$ bis 25 mm$^2$, vorzugsweise von 0,1 mm$^2$ bis 10 mm$^2$, gemäß dem Prüfverfahren hierin aufweist.

14. Tragbarer Artikel (20) nach einem der vorstehenden Ansprüche, wobei, in dem elastischen Laminat, die Porosität der Innenschicht kleiner als die Porosität der Außenschicht gemäß dem Prüfverfahren hierin ist.

15. Tragbarer Artikel (20) nach einem der vorstehenden Ansprüche, wobei, in dem elastischen Laminat, die Innenschicht ein Flächengewicht von 5 g/m$^2$ bis 45 g/m$^2$ aufweist, und die Außenschicht ein Flächengewicht von 10 g/m$^2$ bis 45 g/m$^2$ aufweist.

16. Tragbarer Artikel (20) nach einem der vorstehenden Ansprüche, wobei die zweite Schicht eine Dicke von wenigstens 50 μm, vorzugsweise wenigstens 80 μm, gemäß dem hierin dargelegten Prüfverfahren aufweist.

17. Tragbarer Artikel (20) nach einem der vorstehenden Ansprüche, wobei das elastische Laminat in wenigstens einer Richtung eine Verlängerungsrate von wenigstens 110 % aufweist.

18. Tragbarer Artikel (20) nach einem der vorstehenden Ansprüche, wobei das elastische Laminat als ein Bestandteil verwendet wird, der aus der Gruppe ausgewählt ist, bestehend aus einem elastischen Bund (40), einem Taillenband, einem Seitenfeld, Beinbündchen und einem Außenmantel.

19. Tragbarer Artikel (20) nach einem der vorstehenden Ansprüche, wobei das elastische Laminat durch einen elastischen Bund (40) umfasst wird oder diesen ausbildet, wobei dieser aus einem vorderseitigen Bund (84) und einem rückseitigen Bund (86) ausgebildet ist.

20. Tragbarer Artikel (20) nach Anspruch 18 oder 19, wobei der elastische Bund (84) eine Außenschichtumfaltregion umfasst, wobei die Außenschicht über das elastische Laminat hinaus verlängert ist, und der verlängerte Teil der Außenschicht um das elastische Laminat gefaltet ist, wobei der umgefaltete Teil der Außenschicht dadurch eine dritte Schicht ausbildet, derart, dass wenigstens ein Abschnitt des elastischen Bunds (84) die Innenschicht umfasst, angeordnet zwischen der Außenschicht und der dritten Schicht in Sandwichform, wobei die dritte Schicht, die dem Körper des Trägers in Verwendung zugewandt ist, behandelt ist, um eine gleiche oder eine höhere Hydrophobie als die Innenschicht aufzuweisen.

21. Tragbarer Artikel (20) nach Anspruch 18 oder 19, wobei der elastische Bund (84) eine Innenschichtumfaltregion umfasst, wobei die Innenschicht über das elastische Laminat hinaus verlängert ist, und der verlängerte Teil der Innenschicht selbst umgefaltet ist, wobei der umgefaltete Teil der Innenschicht dadurch eine dritte Schicht ausbildet, derart, dass wenigstens ein Abschnitt des elastischen Bunds (40) die Innen-, die Außen- und die dritte Schicht umfasst, wobei die dritte Schicht, die der Haut des Trägers in Verwendung zugewandt ist, optional behandelt ist, um eine höhere Hydrophobie als die Innenschicht aufzuweisen.

22. Tragbarer Artikel (20) nach Anspruch 21, wobei der elastische Bund (40) ferner eine Außenschichtumfaltregion umfasst, wobei die Außenschicht über das elastische Laminat hinaus verlängert ist, und der verlängerte Teil der Außenschicht um die dritte Schicht gefaltet ist, wobei der umgefaltete Teil der Außenschicht dadurch eine vierte Schicht bildet, derart, dass wenigstens ein Abschnitt des elastischen Bunds (40) die Innenschicht und die dritte Schicht umfasst, angeordnet zwischen der Außenschicht und der vierten Schicht in Sandwichform, wobei sich die dritte Schicht über die vierte Schicht hinaus erstreckt, die Verlängerung der vierten Schicht weniger als 20 % oder weniger als 10 % des Oberflächenbereichs des elastischen Laminats ausbildet, der bestimmt wird, wenn das elastische Laminat derart gedehnt ist, dass die Innen- und die Außenschicht abgeflacht sind.

23. Tragbarer Artikel (20) nach einem der Ansprüche 18 bis 22, umfassend eine mittige Grundeinheit (38) und einen ringartigen elastischen Bund (40), wobei der ringartige elastische Bund (40) aus dem elastischen Laminat ausgebildet ist und aus einem vorderseitigen Bund (84) und einem rückseitigen Bund (86) besteht; die Mitte des vorderseitigen Bundes (84) mit einem vorderseitigen Taillenfeld (52) der mittigen Grundeinheit (38) verbunden ist, die Mitte des rückseitigen Bundes (86) mit einem rückseitigen Taillenfeld (54) der mittigen Grundeinheit (38) verbunden ist und der Rest der mittigen Grundeinheit (38) den Schrittbereich (30) ausbildet, wobei der vorderseitige und der rückseitige Bund (84, 86) jeder ein linkes Seitenfeld und ein rechtes Seitenfeld (82) aufweisen, wo die mittige Grundeinheit (38) nicht überlappt, und die Querränder des vorderseitigen Bundes (84) und des rückseitigen Bundes (86) durch eine Naht verbunden sind, um eine Taillenöffnung (88) und zwei Beinöffnungen auszubilden; wobei der vorderseitige Bund (84) und der rückseitige Bund (86) in dem Schrittbereich (30) in der Längsrichtung zueinander unterbrochen sind; die mittige Grundeinheit (38) umfassend eine Außenmantelschicht (42) auf der Oberfläche, die einem Kleidungsstück zugewandt ist, und eine Unterschicht (60), die an die Innenoberfläche der Außenmantelschicht gebunden ist; wobei die Längslänge der Außenmantelschicht (42) länger als die Längslänge des Schrittbereichs (30) und kürzer als die Längslänge der Unterschicht (60) ist.

**Revendications**

1. Article pouvant être porté (20) comprenant un stratifié élastique, le stratifié élastique comprenant une couche non tissée interne et une couche non tissée externe, dans lequel la couche interne et la couche externe sont directement jointes l'une à l'autre sur une zone d'environ 5 % à environ 50 % en ce sens que la couche interne et la couche externe sont directement fixées l'une à l'autre par l'application d'agents adhésifs ;

la couche interne étant plus proche du corps du porteur que la couche externe lorsque l'article est porté, la couche interne ayant une première surface opposée à la couche externe et une seconde surface tournée vers la couche externe,
la couche externe ayant une hydrophilie plus élevée que la couche interne et ayant une première surface tournée vers la couche interne et une seconde surface opposée à la couche interne, la couche interne étant hydrophobe et la couche externe étant hydrophile ;

le stratifié ayant un calibre total lorsqu'il est soumis au procédé NMR MOUSE tel que défini ici, le calibre total ayant un premier sous-calibre correspondant à 50 % du calibre total en partant de la seconde surface de la couche externe et s'étendant vers la première surface de la couche interne, et un second sous-calibre correspondant aux 50 % restants du calibre total en partant de la première surface de la couche interne et s'étendant vers la seconde surface de la couche externe, et

dans lequel le stratifié élastique a un rapport liquide du premier sous-calibre au second sous-calibre d'au moins 1,1 après 1 minute selon le procédé NMR MOUSE présenté ici ;

dans lequel, dans le stratifié élastique, la masse surfacique de la couche interne n'est pas supérieure à la masse surfacique de la couche externe.

2. Article pouvant être porté (20) selon la revendication 1, dans lequel le stratifié élastique a un rapport liquide du premier sous-calibre au second sous-calibre d'au moins 2,0 après 20 minutes selon le procédé NMR MOUSE présenté ici.

3. Article pouvant être porté (20) selon la revendication 1 ou 2, dans lequel au moins 40 %, ou au moins 50 % de la surface du stratifié élastique, déterminée lorsque le stratifié élastique est étiré de telle sorte que la couche interne et externe sont aplaties, ne comprend aucune autre couche que la couche interne et externe.

4. Article pouvant être porté (20) selon l'une quelconque des revendications précédentes, dans lequel la couche interne et externe ne sont pas élastiques.

5. Article pouvant être porté (20) selon l'une quelconque des revendications précédentes, dans lequel la couche interne est un non-tissé filé-lié, ou un non-tissé cardé-lié par air.

6. Article pouvant être porté (20) selon l'une quelconque des revendications précédentes, dans lequel la couche externe est un non tissé cardé lié par air.

7. Article pouvant être porté (20) selon l'une quelconque des revendications précédentes, dans lequel le stratifié élastique comprend en outre une pluralité de corps élastiques, tels que des fils élastiques et/ou des rubans élastiques, fournis entre la couche interne et externe.

8. Article pouvant être porté (20) selon la revendication 7, dans lequel l'article a un sens longitudinal et un sens transversal, dans lequel, dans le stratifié élastique, les corps élastiques (96) s'étendent le long du sens transversal et sont espacés l'un de l'autre dans le sens longitudinal de l'article.

9. Article pouvant être porté (20) selon la revendication 7 ou 8, dans lequel les corps élastiques (96) et la couche interne et externe sont au moins partiellement joints au moyen d'un adhésif.

10. Article pouvant être porté (20) selon la revendication 7, 8 ou 9, dans lequel les couches interne et externe ainsi que la pluralité de corps élastiques (96) sont au moins partiellement jointes au moyen de la jonction directe des fibres de la première couche et de la seconde couche.

11. Article pouvant être porté (20) selon l'une quelconque des revendications précédentes, dans lequel la couche externe a une pluralité de perforations, telles que des ouvertures, dans la seconde couche à un taux de perforation d'environ 5 % à environ 50 %, de préférence d'environ 5 % à environ 30 %, selon le procédé de test présenté ici.

12. Article pouvant être porté (20) selon la revendication 11, dans lequel la couche externe a une zone de perforation effective de 0,1 mm$^2$ à 25 mm$^2$, de préférence de 0,1 mm$^2$ à 10 mm$^2$, selon le procédé de test présenté ici.

13. Article pouvant être porté (20) selon l'une quelconque des revendications précédentes, dans lequel, dans le stratifié élastique, la couche interne a une pluralité de perforations, telles que des ouvertures, à un taux de perforation de 5 % à 30 %, de préférence de 5 % à 15 %, et la couche interne a une zone de perforation effective de 0,1 mm$^2$ à 25 mm$^2$, de préférence de 0,1 mm$^2$ à 10 mm$^2$, selon le procédé de test présenté ici.

14. Article pouvant être porté (20) selon l'une quelconque des revendications précédentes, dans lequel, dans le stratifié élastique, la porosité de la couche interne est plus petite que la porosité de la couche externe, selon le procédé de test présenté ici.

15. Article pouvant être porté (20) selon l'une quelconque des revendications précédentes, dans lequel, dans le stratifié élastique, la couche interne a une masse surfacique allant de 5 g/m$^2$ à 45 g/m$^2$, et la couche externe a une masse surfacique allant de 10 g/m$^2$ à 45 g/m$^2$.

16. Article pouvant être porté (20) selon l'une quelconque des revendications précédentes, dans lequel la seconde couche a une épaisseur d'au moins 50 $\mu$m, de préférence d'au moins 80 $\mu$m selon le procédé de test présenté ici.

17. Article pouvant être porté (20) selon l'une quelconque des revendications précédentes, dans lequel le stratifié élastique a un taux d'allongement d'au moins 110 % dans au moins un sens.

18. Article pouvant être porté (20) selon l'une quelconque des revendications précédentes, dans lequel le stratifié élastique est utilisé en tant que composant choisi dans le groupe constitué d'une ceinture élastique (40), d'une bande de taille, d'un panneau latéral, de revers de jambe et d'une couverture extérieure.

19. Article pouvant être porté (20) selon l'une quelconque des revendications précédentes, dans lequel le stratifié élastique est compris par, ou forme une ceinture élastique (40) formée d'une ceinture avant (84) et d'une ceinture arrière (86).

20. Article pouvant être porté (20) selon la revendication 18 ou 19, dans lequel la ceinture élastique (84) comprend une région de pliage de couche externe dans lequel la couche externe est allongée au-delà du stratifié élastique, et la partie allongée de la couche externe est repliée sur le stratifié élastique, la partie repliée de la couche externe formant ainsi une troisième couche de telle sorte qu'au moins une partie de la ceinture élastique (84) comprend la couche interne prise en sandwich entre la couche externe et la troisième couche, dans lequel la troisième couche tournée vers le corps du porteur en cours d'utilisation est traitée pour avoir une hydrophobie égale ou supérieure à celle de la couche interne.

21. Article pouvant être porté (20) selon la revendication 18 ou 19, dans lequel la ceinture élastique (84) comprend une région de pliage de couche interne dans lequel la couche interne est allongée au-delà du stratifié élastique, et la partie allongée de la couche interne est repliée sur elle-même, la partie repliée de la couche intérieure formant ainsi une troisième couche de telle sorte qu'au moins une partie de la ceinture élastique (40) comprend la couche interne, la couche externe et la troisième couche, dans lequel la troisième couche tournée vers la peau du porteur en cours d'utilisation est facultativement traitée pour avoir une hydrophobie plus élevée que la couche interne.

22. Article pouvant être porté (20) selon la revendication 21, dans lequel la ceinture élastique (40) comprend en outre une région de pliage de couche externe dans lequel la couche externe est allongée au-delà du stratifié élastique et la partie allongée de la couche externe est repliée sur la troisième couche, la partie repliée de la couche externe formant ainsi une quatrième couche de telle sorte qu'au moins une partie de la ceinture élastique (40) comprend la couche interne et la troisième couche intercalée entre la couche externe et la quatrième couche, dans lequel la troisième couche s'étend au-delà de la quatrième couche, l'extension de la quatrième couche formant moins de 20 %, ou moins de 10 % de la surface du stratifié élastique, déterminée lorsque le stratifié élastique est étiré de telle sorte que la couche interne et externe sont aplaties.

23. Article pouvant être porté (20) selon l'une quelconque des revendications 18 à 22, comprenant un châssis central (38) et une ceinture élastique en forme d'anneau (40), la ceinture élastique en forme d'anneau (40) étant formée du stratifié élastique et constituée d'une ceinture avant (84) et d'une ceinture arrière (86) ; le centre de la ceinture avant (84) étant joint à un pan de taille avant (52) du châssis central (38), le centre de la ceinture arrière (86) étant joint à un pan de taille arrière (54) du châssis central (38), et le reste du châssis central (38) formant une région d'entrejambe (30), la ceinture avant et arrière (84, 86) ayant chacune un pan latéral gauche et un pan latéral droit (82) où le châssis central (38) ne chevauche pas, et les bords transversaux de la ceinture avant (84) et de la ceinture arrière (86) étant joints par une couture pour former une ouverture de taille (88) et deux ouvertures de jambe ; dans lequel la ceinture avant (84) et la ceinture arrière (86) sont mutuellement discontinues dans la région d'entrejambe (30) dans le sens longitudinal ; le châssis central (38) comprenant une couche de recouvrement externe (42) sur la surface tournée vers le vêtement et une feuille de fond (60) liée à la surface interne de la couche de recouvrement externe ; dans lequel la longueur longitudinale de la couche de recouvrement externe (42) est plus longue que la longueur longitudinale de la région d'entrejambe (30) et plus courte que la longueur longitudinale de la feuille de fond (60).

FIG. 1A

**FIG. 1B**

FIG. 2A　　　　FIG. 2B　　　　FIG. 2C

FIG. 3

FIG. 4

FIG. 5A

FIG. 5B

**FIG. 6**

FIG. 7

FIG. 8

FIG. 9

FIG. 10

**FIG. 11**

FIG. 12

FIG. 13

FIG. 14

FIG. 15

**FIG. 16**

**FIG. 17**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017012319 A
- JP 2017113186 A
- JP 2010246901 A
- WO 2017002484 A
- US 2006047260 A
- US 2008287897 A
- WO 2011087503 A
- US 5628097 A
- WO 201673712 A
- WO 200617718 A

**Non-patent literature cited in the description**

- Contact Angle, Wettability and Adhesion. American Chemical Society, 1964
- **NOBUYUKI OTSU.** A Threshold Selection Method from Gray-Level Histograms. *IEEE Transactions on Systems, Man, and Cybernetics,* January 1979, vol. SMC-9 (1
- **T. HILDEBRAND ; P. RUEGSEGGER.** A new method for the model-independent assessment of thickness in three-dimensional images. *Journal of Microscopy,* 1996, vol. 185, 67-75